# EUROPÄISCHE PATENTANMELDUNG

(11) **EP 4 190 192 A1**
(43) Veröffentlichungstag der Anmeldung: **07.06.2023**
(21) Anmeldenummer: 21212220.4
(22) Anmeldetag: 03.12.2021
(51) Int. Cl.: A24F 42/20, B65D 83/20, B65D 83/30, B65D 83/38, B65D 83/42, B65D 83/44, B65D 83/64

(54) **FLÜSSIGKEITSSPENDER UND FLÜSSIGKEITSKARTUSCHEN FÜR EINEN SOLCHEN FLÜSSIGKEITSSPENDER**

(71) Anmelder: Aptar Radolfzell GmbH, 78315 Radolfzell (DE)
(72) Erfinder: Greiner-Perth, Jürgen, 78244 Gottmadingen (DE)
(74) Vertreter: Witte, Weller und Partner Patentanwälte mbB Stuttgart

(57) **Zusammenfassung**

Bekannt sind als Wechselkartusche ausgebildete Flüssigkeitskartuschen zur Aufnahme von unter Druck stehender Flüssigkeit für Flüssigkeitsspender.

Vorgeschlagen wird, dass eine solche Flüssigkeitskartusche (10) einen aus mindestens zwei Wandungsbauteilen (20A, 20B) zusammengesetzten Außenkörper (20) aufweist, der einen Druckraum (22) zur Aufnahme von unter Druck stehendem Gas und unter Druck stehender Flüssigkeit umgibt. Die Flüssigkeitskartusche (10) weist eine Austrittsöffnung (30) auf, die eines der Wandungsbauteile (20A) durchdringt. Die Flüssigkeitskartusche (10) weist darüber hinaus ein Austrittsventilbauteil (40) auf, welches an einer Innenseite des Druckraums (22) an mindestens einem Wandungsbauteil (20A) beweglich gehalten oder befestigt ist und welches in einem geschlossenen Zustand den Austritt von Flüssigkeit aus dem Druckraum (22) durch die Austrittsöffnung (30) unterbindet, wobei das Austrittsventilbauteil (40) durch Kraftbeaufschlagung von außen in einen geöffneten Zustand überführt werden kann.

Zu Trennung von Gas und Flüssigkeit im Druckraum beinhaltet der Druckraum (22) eineverlagerbare oder verformbare Trennwand (52, 64), die den Druckraum (22) in einen Gasraum (24) und einen Flüssigkeitsraum (26) unterteilt.

## Beschreibung

### ANWENDUNGSGEBIET UND STAND DER TECHNIK

Die Erfindung betrifft den Bereich der Flüssigkeitsspender, insbesondere den Bereich der Flüssigkeitsspender zum Austrag von pharmazeutischen Flüssigkeiten oder zum Austrag von Flüssigkeiten aus dem Bereich der Körperhygiene und Parfum sowie den Bereich von Flüssigkeitsspendern zum Austrag von nikotin-oder cannabishaltigen Flüssigkeiten.

Dabei betrifft die Erfindung primär den Bereich von unter Druck stehenden Flüssigkeitskartuschen als Teil solcher Flüssigkeitsspender, in denen die Flüssigkeit vor dem Austrag gelagert ist. Ein solcher Flüssigkeitsspender umfasst neben der Flüssigkeitskartusche eine Austragvorrichtung, in die die Flüssigkeit aus der Flüssigkeitskartusche einströmt und von der aus die Flüssigkeit in eine Umgebung abgegeben wird. Eine Flüssigkeitskartusche gattungsgemäßer und erfindungsgemäßer Art enthält druckbeaufschlagte Flüssigkeit, so dass es keiner Pumpe bedarf, um die Flüssigkeit zum Zwecke der Abgabe in die Austragvorrichtungzu pumpen.

Gattungsgemäße und erfindungsgemäße Flüssigkeitskartuschen sind meist für den Austausch durch den Nutzer ausgebildet und hierfür insbesondere werkzeuglos wechselbar. Die entleerten Flüssigkeitskartuschen werden vom Nutzer entsorgt. Eine neue Flüssigkeitskartusche wird anschließend in die Austragvorrichtung eingefügt oder an dieser angekoppelt.

Die Wiederverwendung der Austragvorrichtung ist in ökologischer Hinsicht von Vorteil. Verbrauchte Flüssigkeitskartuschen verbleiben jedoch als Müll.

### AUFGABE UND LÖSUNG

Aufgabe der Erfindung ist es, eine Gestaltung einer Flüssigkeitskartusche sowie Befüllungstechniken hierfür zu Verfügung zu stellen, die eine einfache Herstellung und eine möglichst wenig umweltbelastende Entsorgung gestatten.

Zur Lösung dieser Aufgabe wird eine Flüssigkeitskartusche zur Aufnahme von unter Druck stehender Flüssigkeit vorgeschlagen, die in nachfolgend beschriebener Weise erfindungsgemäß ausgestaltet ist. Eine solche erfindungsgemäße Flüssigkeitskartusche bildetvorzugsweise zusammen mitderdaran gekoppelten Austragvorrichtung einen erfindungsgemäßen Flüssigkeitsspender. Daneben werden erfindungsgemäß auch Verfahren zur erfindungsgemäßen Befüllung einer solchen Flüssigkeitskartusche vorgeschlagen.

Die erfindungsgemäße Flüssigkeitskartusche weist einen aus mindestens zwei Wandungsbauteilen zusammengesetzten Außenkörper auf, der einen Druckraum umgibt. Vorzugsweise handelt es sich um genau zwei Wandungsbauteile, die den Außenkörper bilden. Die Wandungsbauteile sind über Kopplungsgeometrien oder mittels einer Klebe- oder Schweißverbindung fest und gasdicht miteinander verbunden, wobei die Verbindung der Wandungsbauteile in nachfolgend noch erläuterter Weise vor der Befüllung oder im Zuge der Befüllung stattfinden kann.

Der Außenkörper umgibt den Druckraum, welcher zur Aufnahme von unter Druck stehendem Gas und unter Druck stehender Flüssigkeit in getrennten Räumen vorgesehen ist. Um Flüssigkeit aus dem Druckraum entnehmen zu können, ist eine Austrittsöffnung vorgesehen, die eines der Wandungsbauteile durchdringt. Der Druck im Druckraum drückt die Flüssigkeit durch die Austrittsöffnung, wenn diese geöffnet ist.

Um die Austrittsöffnung vor Verwendung der Flüssigkeitskartusche geschlossen zu halten und vorzugsweise auch nach Entleerung der Flüssigkeitskartusche sicherheitshalber wieder zu verschließen, weist die Flüssigkeitskartusche erfindungsgemäß ein Austrittsventil mit einem Austrittsventilbauteil auf, welches an einer Innenseite des Druckraums an mindestens einem Wandungsbauteil beweglich gehalten oder insbesondere befestigt ist und welches in einem geschlossenen Zustand den Austritt von Flüssigkeit aus dem Druckraum durch die Austrittsöffnung unterbindet. Das Austrittsventilbauteil wird bei der Herstellung üblicherweise an einem der Wandungsbauteile durch dessen offene Seite hindurch befestigt, bevor dieses mit einem zweiten Wandungsbauteil verbunden wird.

Das Austrittsventilbauteil kann durch externe Kraftbeaufschlagung von außen in einen geöffneten Zustand überführt werden, insbesondere durch Einsetzen der Flüssigkeitskartusche in eine hierfür ausgebildete Austragvorrichtung. Hierdurch kann insbesondere ein Öffnungsstutzen in die Austrittsöffnung einfahren und das Austrittsventilbauteil kraftbeaufschlagen, so dass es in seine öffnende Lage verlagert wird. Alternativ kann auch am Austrittsventilbauteil ein durch die Austrittsöffnung hindurch nach außen ragender Betätigungsabschnitt mit einer von außen zugänglichen Betätigungsfläche zum Auslenken und damit Öffnen des Austrittsventilbauteils vorgesehen sein.

Neben der Gestaltung mit einem solchen Austrittsventil ist grundsätzlich auch die Verwendung eines irreversibel zu öffnenden Verschlussorgans möglich, beispielsweise einer festen Membran, die einem Druck von mehreren Bar standhält und beim Einsetzen der Flüssigkeitskartusche in die Austragvorrichtung durch eine Nadel oder eine Klinge der Austragvorrichtung zum Zwecke des Öffnens aufgeschnitten wird.

Der Druckraum einer erfindungsgemäßen Flüssigkeitskartusche beinhaltet eine verlagerbare oder verformbare Trennwand, die den Druckraum in einen Gasraum und einen Flüssigkeitsraum unterteilt. Bei der Trennwand kann es sich insbesondere um eine als Ganzes verschiebliche Trennwand als Teil eines Trennkolbens handeln oder aber um eine als Ganzes oder partiell flexible Wandung, die den Flüssigkeitsraum teilweise und vorzuweise weitgehend umgibt. Im Weiteren ist eine solche flexible Wandung auch als Beutel bezeichnet.

Das Innenvolumen des Druckraums einschließlich Flüssigkeitsraum und Gasraum beträgt vorzugsweise weniger als 200 ml, insbesondere vorzugsweise weniger als 118 ml. Der Flüssigkeitsraum selbst weist im Lieferzustand der befüllten Flüssigkeitskartusche vorzugsweise ein Volumen von weniger als 118 ml auf.

Eine erfindungsgemäße Flüssigkeitskartusche kann aus sehr wenigen Teilen bestehen. Handelt es sich um eine Flüssigkeitskartusche mit Beutel, so weist diese vorzugsweise maximal vier Teile auf, nämlich die beiden Wandungsbauteile, das Austrittsventilbauteil und den Beutel. Wenn das Austrittsventilbauteil und der Beutel einstückig ausgebildet sind, handelt es sich ggf. sogar nur um drei Teile. Bei einer Flüssigkeitskartusche mit Trennkolben sind vorzugsweise genau vier Einzelteile vorgesehen, nämlich die beiden Wandungsbauteile, das Austrittsventilbauteil und ein einstückiger Trennkolben.

Die verlagerbare bzw. verformbare Trennwand führt dazu, dass im Gasraum und der Flüssigkeitsraum vorzugsweise ein identischer oder sehr ähnlicher Druck herrscht. Bei einer Gestaltung mit Trennkolben besteht vorzugsweise im Gasraum ein geringfügig höherer Druck als im Flüssigkeitsraum. Dies wird im Weiteren noch erläutert.

Wird das Austrittsventilbauteil in seinen geöffneten Zustand gebracht, also entsprechend verlagert oder verformt, so sorgt der Druck im Druckspeicher dafür, dass Flüssigkeit durch die Austrittsöffnung hindurch abgegeben wird, während sich der Flüssigkeitsraum entsprechend verkleinert und der Gasraum vergrößert, wobei der Gasdruck abnimmt. Die Gasmenge im Gasraum ist so bemessen, dass die gesamte Flüssigkeit oder zumindest der weit überwiegende Teil der Flüssigkeit (>95%) mit einem ausreichenden Druck ausgetragen werden, vorzugsweise einem Druck von mindestens 2 bar, insbesondere vorzugsweise von mindestens 3 bar. Bei Erstöffnung der Flüssigkeitskartusche beträgt der Druck im Druckraum vorzugsweise mehr als 5 bar, vorzugsweise zwischen 7 bar und 10 bar.

Vorzugsweise bilden zwei Wandungsbauteile den Außenkörper. An einem dieser Wandungsbauteile, vorzugsweise an einem eine erste Stirnwand des Außenkörpers bildenden Wandungsbauteil, ist die Austrittsöffnung vorgesehen, die vorzugsweise zentrisch auf der Stirnwand angeordnet ist. Das andere Wandungsbauteil bildet vorzugsweise eine der Stirnwand gegenüberliegende bodenseitige Wandung. Zum Zwecke der Einbringung von Gas in den Druckraum kann auch hierin eine Öffnung vorgesehen sein. Eine Mantelfläche des Außenkörpers zwischen der Stirnwand und der bodenseitigen Wandungwird vorzugsweise durch das erste der beiden Wandungsbauteile gebildet. Diese Mantelfläche bildet bei einer Gestaltung mit Trennkolben mit ihrer Innenseite eine im Wesentlichen zylindrische Gleitfläche, entlang derer der Trennkolben entlang gleitet. Vorzugweise am unteren Ende der Mantelfläche ist das zweite Wandungsbauteil mit dem ersten Wandungsbauteil umlaufend gasdicht verbunden.

Die geringe Zahl der Teile einer erfindungsgemäßen Gestaltung einer Flüssigkeitskartusche gestattet eine kostengünstige Herstellung. Die wenigen Teile sind darüber hinaus auch Grundlage dafür, dass der Flüssigkeitskartusche eine gute Recyclingfähigkeit aufweist, wie im Weiteren noch erläutert wird.

Das Austrittsventilbauteil, welches allein oder zusammen mit einem der Wandungsbauteile den Ausfluss aus dem Flüssigkeitsraum steuert, kann auf unterschiedliche Arten ausgebildet sein.

Eine mögliche Bauform sieht vor, dass das Austrittsventilbauteil ein frei beweglicher Körper ist, der nicht fest durch die Wandungsbauteile in definierter Lage gehalten wird, sondern stattdessen in einem von einem der Wandungsbauteile gebildeten Ventilraum frei beweglich ist. Vorzugsweise handelt es sich um einen Kugelkörper, insbesondere aus einem thermoplastischen Elastomer (TPE), der in einer durch Überdruck im Flüssigkeitsraum verursachten Endlage eine Ventilöffnung verschließt.

Bevorzugt ist allerdings ein Austrittsventilbauteil, welches stellenweise und insbesondere im Bereich seines Randes fest mit den Wandungsbauteilen verbunden ist, beispielsweise mittels einer Schnappverbindung oder durch ein Einklemmen zwischen mindestens zwei Wandungsbauteilen. Der Austrittsventilbauteil ist in sich verformbar, so dass er trotz der Befestigung in bestimmungsgemäßer Weise partiell beweglich bleibt, um zwischen einem offenen Zustand und einem geschlossenen Zustand verlagerbar zu sein.

Es sind sowohl Austrittsventilbauteile möglich, bei denen das Austrittsventilbauteil selbst eine Ventilöffnung aufweist, die geöffnet und geschlossen werden kann, als auch solche, die zusammen mit einem Wandungsbauteil eine Ventilöffnung bilden.

Bei der erstgenannten Ausgestaltung ist das Austrittsventilbauteil von der Ventilöffnung durchbrochen, wobei an die Ventilöffnung angrenzende Ränder in einem geschlossenen Zustand des Austrittventilbauteils aneinander anliegen und im geöffneten Zustand voneinander zumindest partiell beabstandet sind. Die Ventilöffnung kann dabei insbesondere durch einen Ventilschlitz gebildet sein, der sich durch die Formgebung des Austrittventilbauteils schließt, wenn er nicht mechanisch in eine geöffnete Stellung gedrückt wird. Vorzugsweise ist ein solcher Ventilschlitz an einer zum Flüssigkeitsspeicher hin domartig gewölbten Struktur des Austrittsventilbauteils vorgesehen.

Das Austrittsventilbauteil mit integrierter Ventilöffnung weist vorzugsweise in Richtung des Flüssigkeitsraums weisende Druckflächen auf, die durch Druckbeaufschlagung der Flüssigkeit aufeinander zu gedrückt werden und hierdurch die an die Ventilöffnung angrenzende Ränder zusammendrücken. Der im Druckraum herrschende Druck wirkt somit in Richtung eines geschlossenen Zustandes des Austrittsventilbauteils. Eine mögliche Bauweise dessen sieht einen dünnwandigen Schlauchabschnitt vor, der von außen durch die Flüssigkeit im Flüssigkeitsraum zusammengedrückt wird, so dass er in der sich ergebenden flachen Konfiguration keine Flüssigkeit mehr hindurchtreten lässt, bis er durch ein externes Öffnungsglied aufgeweitet wird.

Das Austrittventilbauteil wird bestimmungsgemäß durch ein externes Öffnungsglied geöffnet, insbesondere einen Füllstutzen während der Befüllung oder durch einen Öffnungsstutzen der Austragvorrichtung während der Verwendung. Damit dieses Öffnen problemlos funktioniert, kann es von Vorteil sein, an einer vom Flüssigkeitsraum weg weisenden Seite des Austrittsventilbauteils eine Aufweitungsstuktur vorzusehen, mittels derer ein einfahrender Füllstutzen die Ventilöffnung aufweiten kann. Es kann sich hierbei um schräggestellte Flächen handeln, insbesondere einander gegenüberliegende schrägstehende Flächen beidseitig eines Ventilschlitzes.

Bei Austrittsventilbauteilen, die zusammen mit einem Wandungsbauteil eine Ventilöffnung bilden, ist vorgesehen, dass das Austrittsventilbauteil durch Anlage an einer Ventilgegenfläche eines der Wandungsbauteile schließt, vorzugsweise durch Anlage an jenem Wandungsbauteil, welches von der Austrittsöffnung durchdrungen ist. Insbesondere kann das Austrittsventilbauteil vor der Austrittsöffnung angeordnet sein und zum Schließen des Ventils umlaufend an einem die Austrittsöffnung innenseitig umgebenden Rand anliegen.

Eine besonders bevorzugte Gestaltung eines solchen Austrittsventilbauteils sieht vor, dass das Austrittsventilbauteil als vorzugsweise im unbelasteten Zustand plane Ventilplatte ausgebildet ist. Hierunter wird ein Austrittsventilbauteil verstanden, welches eine im Wesentlichen einheitliche Dicke aufweist und welches insbesondere vorzugsweise als Stanzteil aus Polyethylen (PE) oder thermoplastischem Elastomer (TPE) hergestellt sein kann, wenngleich auch andere Herstellungsverfahren und/oder Materialien denkbar sind.

Eine solche Ventilplatte ist durch einen von außen einschiebbaren Öffnungsstift oder Öffnungsstutzen auslenkbar, so dass sie ihren geöffneten Zustand einnimmt, indem sie zumindest partiell vom Wandungsbauteil beanstandet wird, an dem sie zuvor umlaufend anlag. Die Ventilplatte ist vorzugsweise umfänglich und insbesondere umlaufend an der Innenseite des Außenkörpers festgelegt, vorzugsweise mittels einer Schnappverbindung.

Damit Flüssigkeit an der Ventilplatte vorbei zur Austrittsöffnung gelangen kann, weist die Ventilplatte vorzugsweise mindestens eine Durchbrechung auf. Gleichzeitig ist die Ventilplatte bei einer solchen Gestaltung vorzugsweise mit einem außenseitigen Rand versehen, der umlaufend an der Innenseite des Außenkörpers anliegt und/oder hier gehalten ist. Alternativ zu der Durchbrechung kann auch vorgesehen sein, dass die Ventilplatte einen außenseitigen Rand aufweist, in dem außenseitig mindestens eine Einbuchtungen vorgesehen ist, im Bereich derer der Rand von der Innenseite des Außenkörpers beabstandet ist. Die Flüssigkeit kann bei einer solchen Gestaltung durch die Einbuchtung hindurch zur Austrittsöffnung gelangen.

Im geschlossenen Zustand des Austrittsventilkörpers liegt dieser vorzugsweise umlaufend um die Austrittsöffnung an der Innenseite des Außenkörpers an. Insbesondere kann ein in RichtungderVentilplatte weisender umlaufender Anlagesteg innenseitig am Außenkörper vorgesehen sein. Dieser Anlagesteg dient als Ventilgegenfläche, an dem die Ventilplatte im geschlossenen Zustand in besonders definierter Weise anliegt. Durch die nur geringe Fläche des Anlagestegs wird eine vergleichsweise hohe Flächenpressung und Dichtigkeit erreicht.

Wie eingangs bereits erläutert, wird die Trennwand zwischen dem Flüssigkeitsraum und dem Gasraum vorzugsweise durch einen Trennkolben oder einen Beutel gebildet.

Wenn die Trennwand durch einen Trennkolben gebildet wird, so ist dieser an einer Innenseite des Außenkörpers entlanggleitend beweglich vorgesehen. Auf der der Austrittsöffnung abgewandten Seite des Trennkolbens ist der Gasraum vorgesehen und auf der der Austrittsöffnung zugewandten Seite des Trennkolbens ist der Flüssigkeitsraum vorgesehen ist.

Der Trennkolben weist die Trennwand umgebend eine zum Gleiten auf einer Innenseite des Außenkörpers geeignete Struktur auf. Vorzugsweise umfasst diese Struktur eine im wesentlichen zylindrische Mantelfläche, die als Gleitfläche dient. Hierdurch kann sich eine napfartige Struktur des Trennkolbens ergeben. Durch diese oder eine anderweitige bezogen auf die Längsachse asymmetrische Struktur kann bewirkt werden, dass bei Überdruck im Flüssigkeitsraum in höherem Maße ein Überfluss in den Gasraum stattfindet als bei Überdruck im Gasraum ein Überfluss in den Flüssigkeitsraum stattfindet. Dies kann in nachfolgend noch beschriebener Weise bei der Befüllung des Druckspeichers genutzt werden, um eine Befüllung des Gasraums durch den Flüssigkeitsraum hindurch zu ermöglichen.

Um eine Befüllung des Gasraums durch den Flüssigkeitsraum hindurch zu erleichtern, kann an der Innenseite des Außenkörpers ein Umströmungskanal vorgesehen sein, der bei Anordnung des Trennkolbens im Bereich des Umströmungskanals ein Umströmen des Trennkolbens ermöglicht. Ein solcher Umströmungskanal ist als örtlich begrenzte Vertiefung in der Gleitfläche vorgesehen. Ist der Trennkolben im Bereich dieser Vertiefung angeordnet, kann einströmendes Gas im Bereich des Trennkolbens in die Vertiefung einströmen und so jenseits des Trennkolbens in den Gasraum gelangen. Ist der Trennkolben später aus der Anfangslage verschoben, so dichtet er im Falle einer Gestaltung mit Umströmungskanal umlaufend weitgehend ab, so dass kein relevantes Umströmen mehr stattfindet.

Es ist bevorzugt, dass die Beweglichkeit des Trennkolbens im Druckspeicher mechanisch begrenzt ist, so dass hierdurch bedingt der Gasraum nicht beliebig verkleinert werden kann. Vorzugweise ist die Begrenzung so gestaltet, dass der Gasraum mindestens 20% des Innenvolumens des Druckraums einnimmt, vorzugsweise mindestens 30%. Eine solche Begrenzung verhindert, dass der Gasraum während der Befüllung des Druckspeichers zu weit reduziert wird und anschließend nicht mehr vergrößert werden kann. Eine bauliche Möglichkeit einer Begrenzung liegt darin, dass an der innenseitigen Gleitfläche der Außenwandung Anschlagserhebungen vorgesehen sind, die vom Trennkolben nicht überfahren werden können. Da dies die Fertigung erschwert, ist vorzugsweise jedoch vorgesehen, dass der Trennkolben und/oder eine bodenseitige Wandung des Außenkörpers mit einem Abstandshalter versehen ist, mittels dessen ein Mindestabstand zwischen der bodenseitigen Wandung und dem Trennkolben gewährleistet wird.

Ist die Trennwand durch einen Beutel gebildet, so ist es die flexible Beutelwand des Beutels, die die Verkleinerung des Flüssigkeitsspeichers ermöglicht. Der Flüssigkeitsraum ist innenseitig der Beutelwand vorgesehen, während der Gasraum zwischen der Beutelwand und dem Außenkörper vorgesehen ist.

Der Beutel kann aus einem biegeschlaffen Material bestehen oder in begrenztem Maße eine elastische Rückstellneigung aufweisen. Insbesondere vorzugsweise kann der Beutel als Tube ausgebildet sein, also ausgehend von einem länglichen Hohlkörper durch endseitiges Zusammendrücken und Verbinden der Ränder gebildet sein.

Die Bauweise der Trennwand als Beutelwand kann vorzugsweise damit einhergehen, dass der Beutelkörper und das Austrittsventilbauteil einstückig ausgebildet sind, so dass die minimale Zahl von Bauteilen der Flüssigkeitskartusche sich auf drei Bauteile reduzieren lässt.

Wie bereits erläutert, liegt die besondere Bedeutung einer erfindungsgemäßen Flüssigkeitskartusche darin, dass diese in Hinblick auf gute Recyclingfähigkeit gestaltet sein kann. Durch geschickte Wahl der Materialien wird erreicht, dass die Wandungsbauteile, die Trennwand und das Austrittsventilbauteil in einem gemeinsamen Recyclingprozess recyclebarsind. Dies bedeutet nicht, dass die Materialien aller Bauteile identisch sein müssen, sondern dass sie sich in einem üblichen Prozess voneinandertrennen lassen. Insbesondere ist für gute Recyclingfähigkeit von Vorteil, wenn die Flüssigkeitskartusche zum überwiegenden Teil aus einem Polyethylenterephthalat (PET) besteht, insbesondere indem die Wandungsbauteile aus PET gefertigt sind. Der Anteil von PET an der Gesamtmasse der entleerten Flüssigkeitskartusche beträgt vorzugsweise mindestens 60%, insbesondere vorzugweise mindestens 75%.

Von PET gutzu trennen sind die leichteren Materialen PE und PP, so dass diese vorzugsweise für den Trennkolben, den Beutelkörper und/oder das Austrittsventilbauteil Verwendung finden. Insbesondere kann PE für den Trennkolben, den Beutel und/oder das Austrittsventilbauteil verwendet werden. Das Austrittsventilbauteil und der Beutel können jedoch auch aus TPE bestehen.

Der Gesamtanteil der genannten drei Materialien PET, PE und PP an der Gesamtmasse der entleerten Flüssigkeitskartusche beträgt vorzugsweise mindestens 75% der Masse der Flüssigkeitskartusche aus diesen Materialien besteht, insbesondere vorzugsweise mindestens 90%.

Bevorzugt ist, wenn alle Bauteile der Flüssigkeitskartusche Materialien aus der Gruppe umfassend Polyethylenterephthalate (PET), Polyethylene (PE), Polypropylene (PP) und thermoplastische Elastomere (TPE) hergestellt sind. Aufgrund der nicht idealen Recyclingfähigkeit von TPE beträgt der Anteil des TPE an der Gesamtmasse der entleerten Flüssigkeitskartusche vorzugsweise weniger als 5%, insbesondere vorzugsweise weniger als 2%.

Eine erfindungsgemäße Flüssigkeitskartusche kann in verschiedenen Arten von Flüssigkeitsspendern Verwendung finden.

So kann die Flüssigkeitskartusche insbesondere mit einer niederviskosen pharmazeutischen Flüssigkeit oder einer der Körperhygiene dienenden Flüssigkeit befüllt sein, wobei insbesondere vorgesehen sein kann, dass der Austrag dieser Flüssigkeit in Form eines Sprühstrahls abgegeben wird.

Ein weiteres Feld, für das sich die Flüssigkeitskartusche eignet, ist das Feld der Flüssigkeitsspender zum Austrag einer nikotin- und/oder cannabishaltigen Flüssigkeit, insbesondere zur Rauchentwöhnung. Auch eine solche Flüssigkeit wird vorzugsweise in Form eines Sprühstrahls abgegeben, insbesondere bei geringem Flüssigkeitsstrom.

Aber auch für hochviskose Flüssigkeiten wie Hautpflegecreme oder Zahnpasta ist eine erfindungsgemäße Flüssigkeitskartusche gut geeignet. Durch den erzielbaren Druck von anfänglich über 6 bar kann erreicht werden, dass selbst im Falle eingetrockneter Reste der Flüssigkeit im Bereich der Austrittsöffnung ein Austrag gewährleistet werden kann. Gegenüber einem Pumpsystem ist eine wesentlich größere Druckdifferenz zwischen dem Flüssigkeitsraum und einer Umgebung mit Normaldruck erzielbar.

Vorzugsweise ist der Flüssigkeitsraum mit einer der genannten Flüssigkeiten befüllt. Der Gasraum ist mit einem komprimierten Gas gefüllt, wobei es sich vorzugsweise um Luft handelt.

Wie eingangs bereits erläutert, sind die mindestens zwei Wandungsbauteile miteinander fest und gasdicht verbunden. Vorzugsweise sind sie miteinander über eine Klebeverbindung oder eine Schweißverbindung verbunden, wobei die Schweißverbindung vorzugsweise eine Laserschweißverbindung, eine Reibschweißverbindung oder eine Ultraschallschweißverbindung sein kann.

Daneben kommt jedoch auch eine Verbindungstechnik in Frage, bei der zwei Wandungsbauteile unter Bildung einer abdichtenden Presspassung miteinander verbunden sind. Vorzugsweise ist in einem solchen Falle eine Kopplungseinrichtung vorgesehen, die eine Gewindeverbindung mit Außengewinde und Innengewinde an den beiden Wandungsbauteilen umfasst. Diese gestattetes, die Wandungsbauteile mit hoher Kraft aneinander anzupressen, um die Gasdichtigkeit zu erzielen. Insbesondere von Vorteil ist es, wenn mindestens eines der Wandungsbauteile mit einer konischen Aufweitung oder Einschnürung versehen ist, um im Zuge des Einschiebens oder Aufschiebens des anderen Wandungsbauteils die Presspassung beim Aufschrauben zu erzielen.

Um die Flüssigkeitskartusche an einer Austragvorrichtung befestigen zu können und damit ein Öffnen des Austrittsventils zu ermöglichen, können passende Kopplungsmittel vorgesehen sein. Insbesondere kann an einer Außenseite des Außenkörpers und insbesondere an dessen Mantelfläche ein Außengewinde oder ein Bajonett zur Ankopplung an eine Austragvorrichtung vorgesehen sein. An einer Innenseite eines Aufnahmeraums der Austragvorrichtung sind korrespondierende Kopplungsmittel vorzusehen.

Es ist bevorzugt, dass während der Befüllung einer erfindungsgemäßen Flüssigkeitskartusche das Gas durch die Austrittsöffnung in den Druckraum gelangt. Allerdings sind auch Bauweisen möglich, bei denen in mindestens einem der Wandungsbauteile eine separate Gasbefüllungsöffnung vorgesehen ist, die unter Umgehung des Flüssigkeitsraums in den Gasraum mündet. Vorzugsweise weist das betreffende Wandungsbauteil eine Ventileinrichtung auf, die durch Überdruck im Gasraum geschlossen wird. Auch ein Stopfenbauteil zum Verschließen der Gasbefüllungsöffnung ist möglich.

Es wird bevorzugt, dass mindestens eines der Wandungsbauteile, insbesondere vorzugsweise alle Wandungsbauteile, aus einem transparenten Kunststoff bestehen, insbesondere PET. Hierdurch wird es möglich, den Füllstand der Flüssigkeitskartusche einfach zu prüfen.

Wie eingangs bereits erläutert, umfasst die Erfindung auch einen Flüssigkeitsspender zum Austrag von Flüssigkeit. Dieser umfasst eine Austragvorrichtung und eine an oder in der Austragvorrichtung befestigte Flüssigkeitskartusche der beschriebenen Art. Die Kopplung der Flüssigkeitskartusche an die Austragvorrichtung erfolgt dabei in lösbarer Weise, damit die Flüssigkeitskartusche vorzugsweise werkzeuglos nach Entleerung durch eine neue ersetzt werden kann.

Die Flüssigkeitskartusche kann vollständig oder weitgehend in einen Aufnahmeraum der Austragvorrichtung eingeschoben sein, so dass sie von außen nicht mehr oder kaum noch zu sehen ist. Vorzugsweise ist an einer Innenseite einer Mantelwandung des Aufnahmeraums ein Innengewinde oder ein Bajonett zur Kopplung mitder Flüssigkeitskartusche vorgesehen. Es ist bevorzugt, dass die Mantelwandung transparent ausgestaltet ist, damit das Vorhandensein einer Flüssigkeitskartusche im Aufnahmeraum und gegebenenfalls deren Füllstand einfach kontrolliert werden kann.

Alternativ zu einem solchen Aufnahmeraum kann jedoch auch vorgesehen sein, dass die Flüssigkeitskartusche zum überwiegenden Teil nach außen freiliegt und somit selbst einen Teil der Außenfläche des Flüssigkeitsspenders bildet.

Vorzugsweise weist die Austragvorrichtung zum Öffnen des Austrittsventils einen Öffnungsstift oder einen Öffnungsstutzen auf, der durch die Austrittsöffnung in die Flüssigkeitskartusche hineinragt und dort auf das Austrittsventilbauteil wirkt. Der Öffnungsstift bzw. Öffnungsstutzen kann dabei zum permanenten oder bedarfsweisen Öffnen des Austrittventils vorgesehen sein.

Eine bevorzugte Bauform mit einer permanenten Öffnung des Austrittsventils durch das den Öffnungsstift oder Öffnungsstutzen sieht vor, dass die Austragvorrichtung zusätzlich zum Austrittsventilbauteil ein mittels einer Betätigungsfläche manuell schaltbares Auslassventil stromabwärts des Austrittsventils aufweist. Es ist bei einer solchen Gestaltung also vorgesehen, dass das Austrittsventil der Flüssigkeitskartusche durch die Austragvorrichtung dauerhaft geöffnet wird und der Flüssigkeitsdruck dann am Auslassventil der Austragvorrichtung anliegt. Dieses muss entsprechend geeignet ausgelegt werden, um gegen den Druck des Druckspeichers zu schließen.

Alternativ kann vorgesehen sein, dass die Austragvorrichtung eine Betätigungsfläche zur manuellen Betätigung aufweist, wobei diese Betätigungsfläche mit einem Öffnungsstift oder Öffnungsstutzen gekoppelt ist, der bei Betätigung eine Kraftbeaufschlagung und damit ein Öffnen des Austrittsventilbauteils bewirkt. Bei einer solchen Gestaltung ist ein zweites Ventil somit nicht erforderlich, da bei Betätigung der Austragvorrichtung unmittelbar das Austrittsventil der Flüssigkeitskartusche öffnet und dieses mit Ende der Betätigung der Betätigungsfläche wieder schließt, insbesondere bedingt durch den Flüssigkeitsdruck im Flüssigkeitsraum.

Der genannte Öffnungsstutzen weist vorzugsweise eine hülsenförmige oder rohrförmige Form auf, wobei die Außenseite des Öffnungsstutzens vorzugsweise einen Durchmesser aufweist, der radial dichtend an einer Innenseite der Austrittsöffnung anliegt.

Wie bereits erwähnt, eignen sich die Flüssigkeitskartusche und die Austragvorrichtung für verschiedene Flüssigkeiten und Applikationsformen, beispielsweise auch für den Austrag hochviskoser Flüssigkeiten. Bevorzugt ist jedoch eine Gestaltung, bei der die Flüssigkeit in Form eines zerstäubten Sprühstrahls abgegeben wird. Um dies zu erreichen, ist die Austragvorrichtung vorzugsweise mit einerentsprechend ausgebildeten Auslassöffnungversehen, beispielsweise mit einer vorgeschalteten Wirbelkammer. Bevorzugt ist eine Gestaltung, bei der die Austragvorrichtung eine Austragdüseneinrichtung umfassend eine Düsenplatte mit mindestens einer Düsenöffnung aufweist. Vorzugsweise weist die Austragdüseneinrichtung eine Mehrzahl von Austrittsdüsenöffnungen auf. Die Düsenöffnungen weisen an ihrer engsten Stelle vorzugsweise einen lichten Querschnitt zwischen 10 µm² und 200 µm² auf, insbesondere vorzugsweise zwischen 40 µm² und 100 µm². Vorzugsweise sind mindestens 20 Düsenöffnungen vorgesehen. Die Austragdüseneinheit, insbesondere die Düsenplatte, weist vorzugsweise Düsenöffnungen auf, die feine Einzelstrahlen erzeugen, welche nach Austritt in Einzeltröpfchen zerfallen, insbesondere in Form eines Zerfalls ohne Lufteinfluss (Rayleigh-Zerfall).

Eine erfindungsgemäße Flüssigkeitskartusche wird vor Auslieferung an den Endkunden mit Gas und Flüssigkeit befüllt. In der Praxis findet zumindest die Befüllung mit Flüssigkeit meist nicht am Ort der Herstellung der Komponenten der Flüssigkeitskartusche statt, sondern an einem für die Befüllung eingerichteten Standort. Die Gasbefüllung kann sowohl am Herstellungsort der Flüssigkeitskartusche oder bei genanntem Befüller stattfinden.

Ausgehend von dem noch leeren Spender, in dem üblicherweise anfänglich Luft unter Normaldruck eingeschlossen ist, erfolgtdie Befüllung mit Gas, insbesondere mit Luft, sowie mit Flüssigkeit, wobei verschiedene Befüllungsverfahren verschiedene Reihenfolgen vorsehen. Auch die gleichzeitige Befüllung mit Gas und Flüssigkeit ist möglich, wenn die Flüssigkeitskartusche neben der Austrittsöffnung eine Gasbefüllungsöffnung aufweist.

Es gibt verschiedene Varianten, mittels derer das Gas durch eine von der Austrittsöffnung abweichende Öffnung in den Gasraum einbracht wird. Eine dieser Varianten sieht vor, dass das Gas durch eine Öffnung eines Wandungsbauteils in den Gasraum eingebracht wird, bevor die Öffnung durch Verbindung des Wandungsbauteils mit einem zweiten der Wandungsbauteile verschlossen wird. Dies bedeutet, dass die mindestens zwei Wandungsbauteile vor der Gasbefüllung noch voneinander getrennt sind.

Alternativ kann zur Gasbefüllung die bereits beschriebene und hierfür vorgesehene Gasbefüllungsöffnung verwendet werden. Dementsprechend können bereits vor Gasbefüllung die Wandungsbauteile miteinander verbunden sein. Damit das Gas nach Befüllung nicht wieder durch die Gasbefüllungsöffnung entweicht, schließt anschließend ein integriertes Gasbefüllungsventil oder die Gasbefüllungsöffnung wird mittels eines Stopfenbauteils verschlossen.

Eine grundsätzlich andere Form der Befüllung sieht vor, dass sowohl das Gas als auch die Flüssigkeit nacheinander durch die Austrittsöffnung hindurch in den Druckraum eingebracht werden. Bei diesem Verfahren kann die Flüssigkeitskartusche entsprechend vor dem Befüllen bereits vollständig zusammengesetzt sein.

Ein Weg zur Befüllung das Gasraums mit Gas und des Flüssigkeitsraums mit Flüssigkeit durch die gleiche Austrittöffnung sieht vor, dass zunächst das Gas in den Druckraum eingebracht wird, wobei es in den Flüssigkeitsraum und am Trennkolben vorbei in den Gasraum gelangt.

Vorzugsweise ist hierfür ein Umströmungskanal der oben beschriebenen Art vorgesehen.

Alternativ ist der Trennkolben derart gestaltet, dass er bei Überdruck im Flüssigkeitsraum das Vorbeiströmen von Gas in geringem Umfang gestattet. Nach Einbringung des Gases wird anschließend in einer Lage der Flüssigkeitskartusche mit unten angeordnetem Flüssigkeitsraum die Flüssigkeit von unten in den Flüssigkeitsraum eingebracht. Dabei verdrängen die Flüssigkeit und ihr Druck das Gas aus dem Flüssigkeitsraum in den Gasraum hinein.

Obwohl gemäß dem beschriebenen Verfahren vorgesehen sein kann, dass der Gasraum am Trennkolben vorbei mit Gas befüllt wird, bedeutet dies nicht, dass auch nach Befüllung mit Gas und Flüssigkeit ein Austausch zwischen dem Gasraum und dem Flüssigkeitsraum durch einen schmalen umgebenden Spalt am Trennkolben stattfindet. Zum einen wird dies durch die Oberflächenspannung der Flüssigkeit verhindert. Zum anderen kann derTrennkolben durch die beschriebene napfartige Formgebung dazu neigen, sich aufzuweiten, sobald der Druck im Gasraum höher als im Flüssigkeitsraum ist. Eine solche Situation stellt sich ein, wenn Flüssigkeit aus dem Flüssigkeitsraum entnommen wird, da der Trennkolben bei passender Auslegung aufgrund seiner umfänglichen Reibung nicht jeden minimalen Druckunterschied zwischen dem Gasraum und dem Flüssigkeitsraum ausgleicht und daher der Druck im Gasraum leicht gegenüber dem Flüssigkeitsraum erhöht bleibt.

Da bei der Befüllung mit Gas die Gefahr besteht, dass der Trennkolben bis in seine der Austrittsöffnung abgewandte Endlage gedrückt wird und der Gasraum dadurch zu sehr verkleinert wird, kann die oben beschriebene Anschlagsfläche oder der oben beschriebene Abstandshalter Verwendung finden, um die Verkleinerung der Gasraums bei der Befüllung zu limitieren.

Sofern der Trennkolben bei der Befüllung des Druckraums mit Gas in einer definierten Stellung verbleiben soll, beispielsweise in einer Stellung, in der er über den Umströmungskanal den Zufluss von Gas in den Gasraum begünstigt, können Haltemittel wie eine Einschnürung, Rasten oder dergleichen vorgesehen sein. Um den Trennkolben nach Gasbefüllung aus der genannten Stellung heraus verschieben zu können, kann ein Gasdruckstoß oder Flüssigkeitsdruckstoß genutzt werden. Auch ist es möglich, dass im Zuge der Befüllung ein Werkzeugs durch die Austrittsöffnung hindurch eingeschoben wird, welches bis in den Bereich des Trennkolbens gelangt und diesen unmittelbar von der Austrittsöffnung weg kraftbeaufschlagt.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt ein erstes Ausführungsbeispiel einer erfindungsgemäßen Flüssigkeitskartusche.
Fig. 2 zeigt die Flüssigkeitskartusche der Fig. 1 in zerlegter Form.
Fig. 3 zeigt in vergrößerter Darstellung das Austrittsventil der Flüssigkeitskartusche der Fig. 1.
Fig. 4 bis 6 zeigen verschiedene Austrittsventilkörper, die bei der Flüssigkeitskartusche der Fig. 1 Verwendungfinden können.
Fig. 7 zeigt eine alternative Bauweise eines Austrittsventils.
Fig. 8 zeigt nochmals das Austrittsventil der Flüssigkeitskartusche der Fig. 1.
Fig. 9 bis 11 zeigen weitere Alternativbauweisen.
Fig. 12 zeigt neben der Flüssigkeitskartusche der Fig. 9 eine Austragvorrichtung, mit der die Flüssigkeitskartusche zur Bildung eines Flüssigkeitsspenders kombiniert wird.
Fig. 13A und 13B zeigen den zusammengesetzten Flüssigkeitsspender im unbetätigten und im betätigten Zustand.
Fig. 14 zeigt eine andere Austragvorrichtung, mit der die Flüssigkeitskartusche der Fig. 1 zu einem Flüssigkeitsspender verbunden ist.
Fig. 15 zeigt ein zweites Ausführungsbeispiel einer erfindungsgemäßen Flüssigkeitskartusche.
Fig. 16 zeigt die Flüssigkeitskartusche der Fig. 15 in zerlegter Form.
Fig. 17 zeigt die eine Variante der Flüssigkeitskartusche der Fig. 15 mit einer separaten Gasbefüllungsöffnung.
Fig. 18 zeigt neben der Flüssigkeitskartusche der Fig. 15 eine Austragvorrichtung, mit der die Flüssigkeitskartusche zur Bildung eines Flüssigkeitsspenders kombiniert wird.
Fig. 19A und 19B zeigen den zusammengesetzten Flüssigkeitsspender im unbetätigten und im betätigten Zustand.
Fig. 20A bis 20D zeigen ein erstes Befüllungsverfahren zur Befüllung einer Flüssigkeitskartusche entsprechend Fig. 1.
Fig. 201 bis 21E zeigen ein zweites Befüllungsverfahren zur Befüllung einer Flüssigkeitskartusche entsprechend Fig. 9.

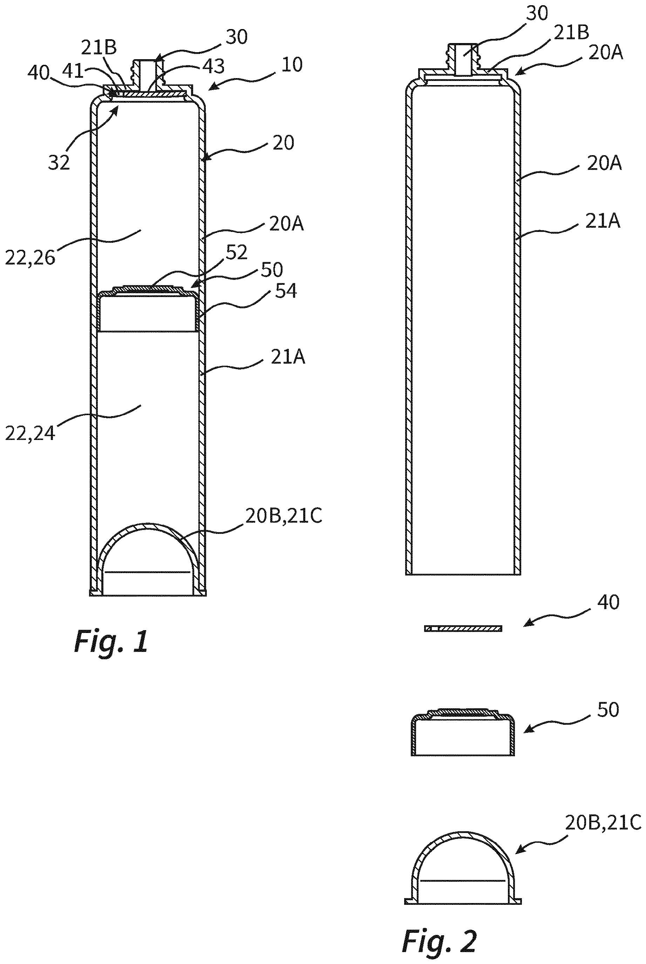

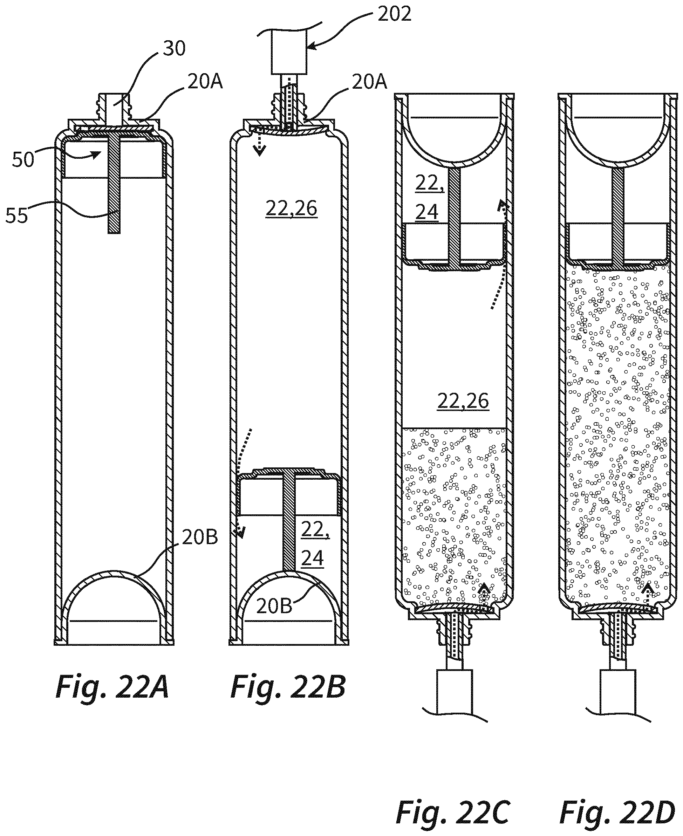

Fig. 22A bis 22D zeigen ein drittes Befüllungsverfahren zur Befüllung einer Flüssigkeitskartusche entsprechend Fig. 1.

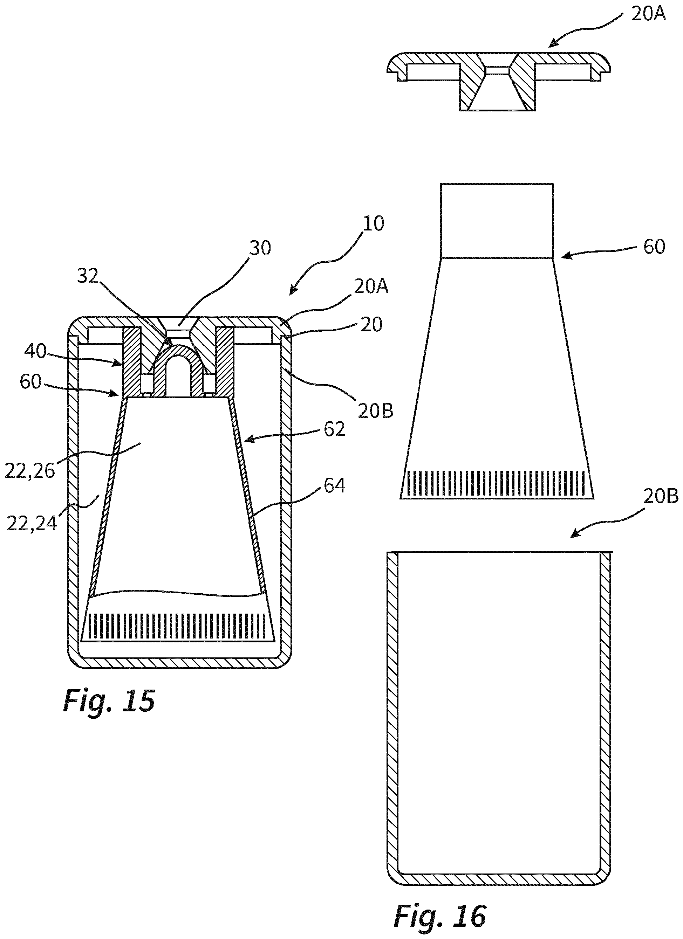

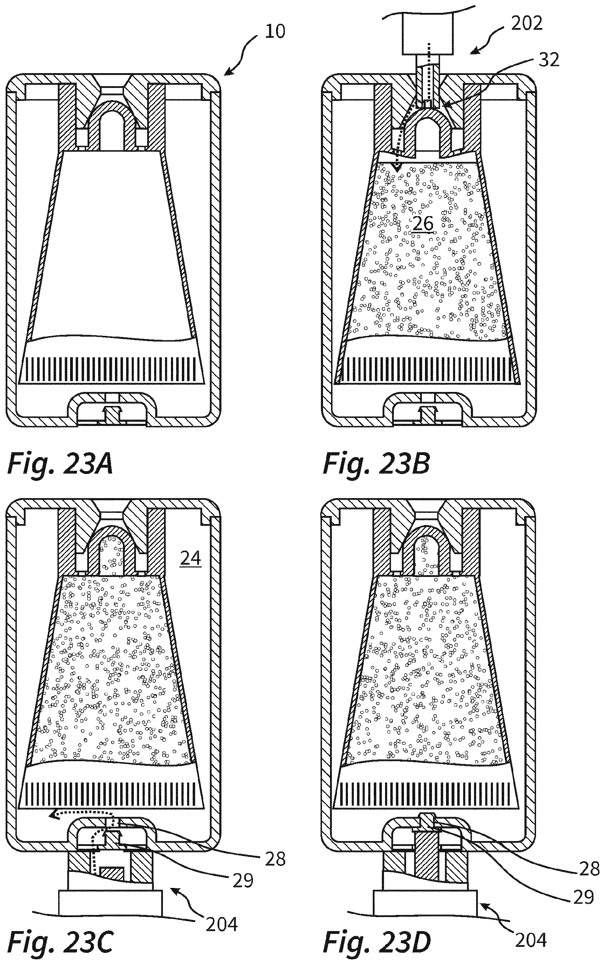

Fig. 23A bis 23D zeigen ein viertes Befüllungsverfahren zur Befüllung einer Flüssigkeitskartusche entsprechend Fig. 15.

### DETAILLIERTE BESCHREIBUNG DERAUSFÜHRUNGSBEISPIELE

Fig. 1 zeigt ein erstes Ausführungsbeispiel einer erfindungsgemäßen Flüssigkeitskartusche 10.

Die Flüssigkeitskartusche 10 verfügt über einen Außenkörper 20 aus PET. Dieser Außenkörper 20 setzt sich aus zwei Wandungsbauteilen 20A, 20B zusammen. Das Wandungsbauteil 20A bildet dabei eine Mantelwand 21A sowie eine obere Stirnwand 21B, im Bereich derer eine Austrittsöffnung 30 vorgesehen ist. Das Wandungsbauteil 20B bildet eine bodenseitige Stirnwand 21C, die eine gewölbte Form aufweist und im zusammengesetzten Zustand der Fig. 1 mit dem ersten Wandungsbauteil 20A gasdicht verbunden ist, insbesondere durch eine Schweißverbindung oder eine Klebeverbindung. Gemeinsam umgeben die den Außenkörper 20 bildenden Wandungsbauteile 20A, 20B einen Druckraum 22, der mittels eines Trennkolbens 50 in einen Gasraum 24 und einen Flüssigkeitsraum 26 unterteilt ist. Der Trennkolben 50 weist neben der eigentlichen Trennwand 52 einen Mantelabschnitt 54 auf, der gleitend entlang einer Innenseite der Mantelwand 21A verlagerbar ist.

Der Austrittsöffnung 30 vorgeschaltet ist ein Austrittsventil 32, welches beim Ausführungsbeispiel der Fig. 1 durch eine im Wesentlichen plane Ventilplatte 40 als Austrittsventilbauteil 40 gebildet wird, die umlaufend randseitig durch eine Schnappverbindung mit dem Wandungsbauteil 20A verbunden ist. Damit Flüssigkeit an der Ventilplatte 40 vorbeiströmen kann, ist diese mit einer Durchbrechung 41 versehen.

Der Fig. 2 sind nochmals die insgesamt vier Einzelbauteile der Flüssigkeitskartusche 10 zu entnehmen. Die beiden Wandungsbauteile 20A, 20B sind vorzugsweise aus transparentem PET gefertigt. Die Ventilplatte 40 besteht vorzugsweise aus thermoplastischem Elastomer (TPE). Der Trennkolben 50 besteht vorzugsweise aus PP. Während der Montage werden die Bauteile 20B, 40, 50 von unten in das Wandungsbauteil 20A eingefügt.

Durch die genannte Materialwahl kann nach der Entsorgung der zuvor entleerten Flüssigkeitskartusche 10 diese sehr gut recycelt werden. Hierzu wird in üblicher Art und Weise die Flüssigkeitskartusche 10 als Ganzes geschreddert, so dass sich ihre Einzelteile vollständig voneinander trennen. Das TPE des Austrittsventilbauteils stellt eine vernachlässigbare Verunreinigung beim Recycling dar, nimmtjedoch bezogen auf die Masse nur etwa 0,5% der Masse der vier Komponenten der Fig. 2 ein. Die voneinander durch das Schreddern gelösten Teile können aufgrund der spezifischen Dichte von PP, die geringer als die von Wasser ist, und von PET, die höher als die von Wasser ist, einfach mittels Schwimm-Senk-Trennung voneinander getrennt werden.

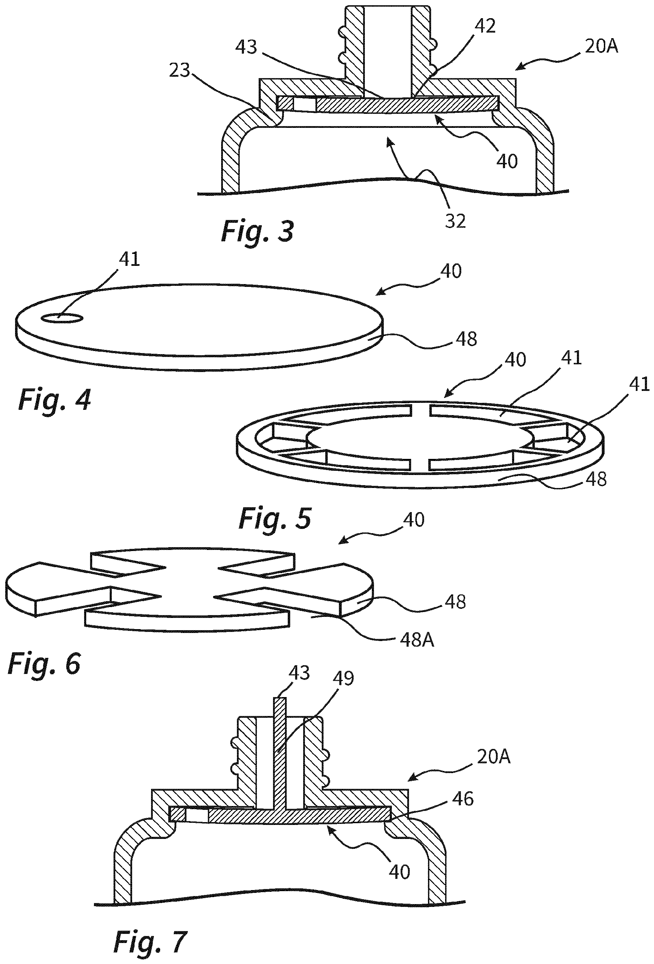

Die Fig. 3 bis 6 verdeutlichen die Bauweise des Austrittventils 32 und Alternativen hierzu. In Fig. 3 ist das Austrittventil 32 vergrößert dargestellt. Es ist zu ersehen, dass das als Ventilplatte 40 ausgebildete Austrittsventilbauteil 40 randseitig durch Schnappvorsprünge 23 gehalten ist. In dem geschlossenen Zustand der Fig. 3 ist das Austrittventil 32 geschlossen. Dies wird dadurch erzielt, dass die Ventilplatte 40 unter leichter Vorspannung mit ihrer Oberseite an einem umlaufenden Anlagesteg 42 im Bereich des Eingangs der Austrittsöffnung 30 anliegt. Da der Flüssigkeitsdruck aufgrund der Flächenverhältnisse die Ventilplatte stärker nach oben als nach unten drückt, wirkt er in SchließrichtungdesAustrittventils 32. Das Öffnen des Austrittventils 32erfolgt dadurch, dass durch die Austrittsöffnung 30 hindurch ein Öffnungsstift oder Öffnungsstutzen eingeschoben wird, der im Bereich einer Betätigungsfläche 43 auf die Ventilplatte drückt, dadurch die die Krümmung der Ventilplatte 40 vergrößert und den Spalt zwischen dem Anlagesteg 42 und der Ventilplatte 40 öffnet. In diesem Zustand ist das Austrittventil 32 geöffnet und die druckbeaufschlagte Flüssigkeit kann austreten.

Die Ventilplatte 40 der Fig. 3 ist in der Fig. 4 nochmals separat dargestellt.

Fig. 5 zeigt eine alternative Bauweise, bei der mehrere Durchbrechungen 41 vorgesehen sind. Fig. 6 zeigt eine Bauweise, bei der keine Durchbrechung 41 vorgesehen ist, sondern stattdessen randseitige Einbuchtungen 48A, die ebenfalls ermöglichen, dass Flüssigkeit durch die Ventilplatte 40 hindurchströmt und somit an der Ventildichtstelle des Anlagestegs 42 anliegt, bis das Austrittsventil 32 geöffnet wird.

Die Ventilplatten der Fig. 4 bis 6 können vorzugsweise als Stanzteile aus TPE oder PP hergestellt sein. Die Herstellung als Stanzteil gewährleistet eine besonders kostengünstige Produktion.

Fig. 7 zeigt eine Alternative, bei der das Austrittsventilbauteil nicht als plane Ventilplatte ausgebildet ist, sondern mit einem Betätigungsstift 49 versehen ist, der durch die Austrittsöffnung 30 hindurch nach außen ragt. Wird dieser Betätigungsstift 49 durch Einfügen der Flüssigkeitskartusche 10 in eine Austragvorrichtung eingedrückt, so drückt dies auf die Betätigungsfläche 43 am außenliegenden Ende des Betätigungsstiftes 49 und öffnet hierdurch das Austrittventil 32.

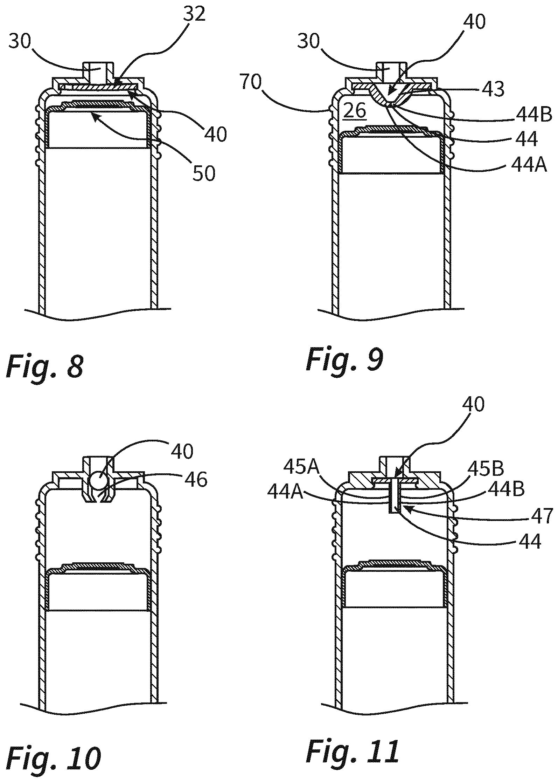

Die Fig. 9 bis 11 zeigen weitere Gestaltungen des Austrittsventilbauteils 40. In Fig. 8 ist nochmals die Gestaltung der Fig. 1 verdeutlicht.

Bei dem Austrittsventilbauteil 40 der Fig. 9 ist dieses aus einem elastischen Kunststoff gefertigt und weist eine in Richtung des Flüssigkeitsraums 26 weisende domartige Erhebung auf. An deren Scheitel ist eine Ventilöffnung 44 in Form eines Ventilschlitzes 44 vorgesehen, der durch zwei Ränder 44a, 44b begrenzt wird, die zum Schließen des Ventils aneinander anliegen. Aufgrund der domartigen Struktur wirkt der Flüssigkeitsdruck im Flüssigkeitsraum 26 derart auf Druckbeaufschlagungsflächen 45A, 45B, dass das Ventil durch den Druck in seine geschlossene Stellung gedrückt wird. Auf der dem Flüssigkeitsraum 26 abgewandten Seite sind schräg gestellte Flächen als Betätigungsflächen 43 vorgesehen, um das Öffnen des Austrittsventils 32 zu erleichtern. Dies wird im Weiteren noch erläutert.

Fig. 10 zeigt eine Gestaltung, bei der das Austrittsventilbauteil 40 nicht am Wandungsbauteil 20A befestigt ist, sondern in einem Ventilraum 46 begrenzt frei beweglich ist. Der Flüssigkeitsdruck im Flüssigkeitsraum 26 drückt das Austrittsventilbauteil 40 in Form einer TPE-Kugel in die Stellung der Fig. 10, in der diese die Austrittsöffnung 30 verschließt. Wird durch einen Öffnungsstift von außen die Kugel nach unten gedrückt, so öffnet das Ventil.

Bei der Gestaltung gemäß Fig. 11 ist vorgesehen, dass das Austrittsventilbauteil 40 einen Schlauchabschnitt 47 aufweist, der aus einem dünnwandigen Material gefertigt ist. Dies führt dazu, dass der Flüssigkeitsdruck im Flüssigkeitsraum 26 diesen Schlauch in horizontaler Hinsichtzusammendrückt und hierdurch das Austrittventil 32 schließt. Um das Austrittventil 32 zu öffnen, muss durch die Austrittsöffnung 30 hindurch ein Öffnungsstift oder Öffnungsstutzen in den Schlauchabschnitt 47 eingeschoben werden.

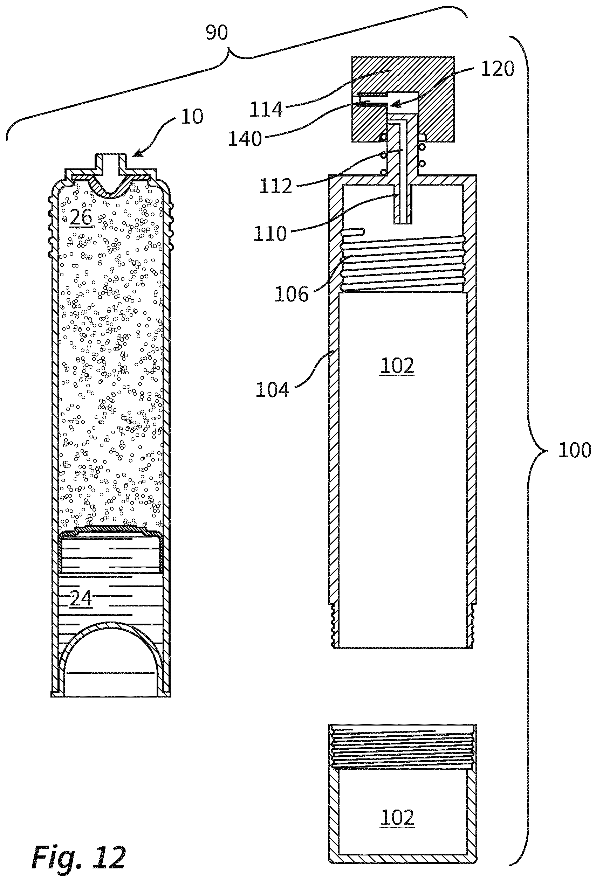

Fig. 12 zeigt die beschriebene Flüssigkeitskartusche 10 mit dem Ventil gemäß Fig. 9 zusammen mit einer Austragvorrichtung 100, mit der zusammen die Flüssigkeitskartusche 10 einen funktionstauglichen Flüssigkeitsspender 90 bildet. Die Austragvorrichtung 100 verfügt über einen Aufnahmeraum 102 sowie einen Öffnungsstutzen 110, der in diesen Aufnahmeraum 102 hineinragt. Der Öffnungsstutzen 110 umgibt einen Auslasskanal 112, der zu einem niederdrückbaren Betätigungsdrücker 114 führt. Der Betätigungsdrücker 114 ist mit einem Austragventil 120 versehen, welches durch Niederdrücken des Betätigungsdrückers 114 geöffnet wird, so dass die Flüssigkeit dann durch eine Austragdüseneinrichtung 140 ausströmen kann.

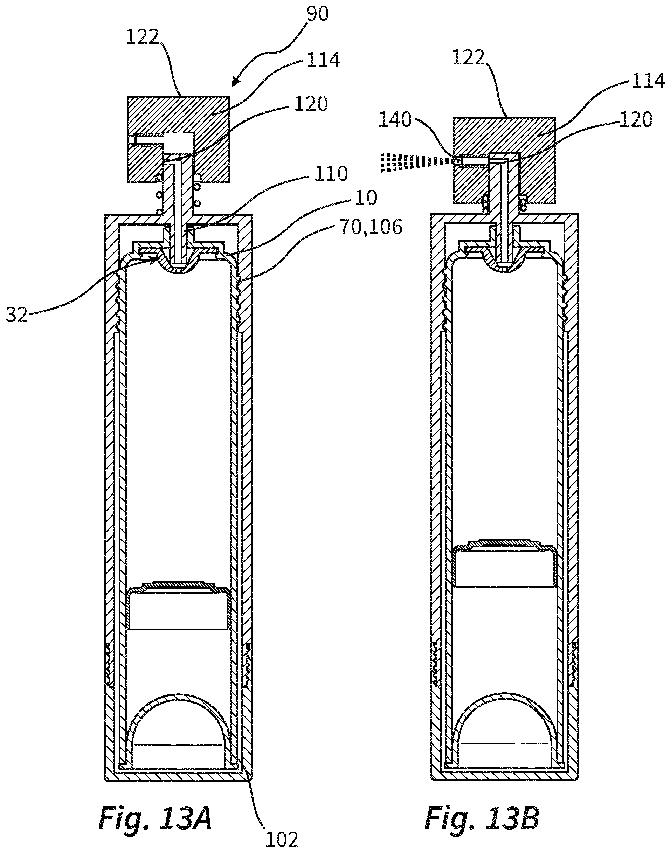

Fig. 13A zeigt die Situation, die sich einstellt, wenn die Flüssigkeitskartusche 10 in den Aufnahmeraum 102 der Austragvorrichtung 100 eingefügt ist. Es ist zu ersehen, dass die Flüssigkeitskartusche 10 mittels eines Außengewindes 70 am Außenkörper 20 in ein korrespondierendes Innengewinde 106 der Austragvorrichtung 100 eingeschraubt ist, so dass der Öffnungsstutzen 110 das Austrittventil 32 geöffnet hat. In diesem Zustand der Fig. 13A steht somit der Flüssigkeitsdruck des Flüssigkeitsraums 26 am Austragventil 120 an. Wird der Betätigungsdrücker 114 mittels seiner Betätigungsfläche 122 niedergedrückt, so gelangtdie Flüssigkeit unter Druck zur Austragdüseneinrichtung 140, wo sie durch feine Düsenöffnungen gepresst wird und somit feine Flüssigkeitsstrahlen bildet, die jenseits der Austragdüseneinrichtung 140 zu Einzeltropfen zerfallen. Dies ist in Fig. 13B dargestellt.

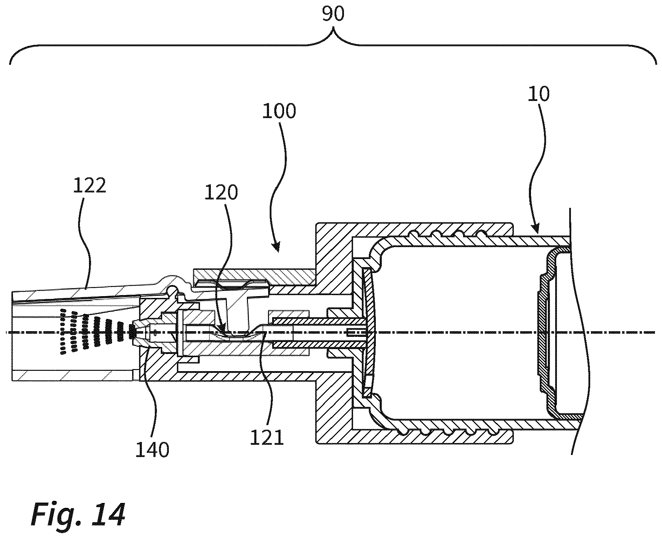

Fig. 14 zeigt eine zweite Austragvorrichtung 100 zur Ankopplung einer Flüssigkeitskartusche 10. Bei dieser Gestaltung handelt es sich um einen Flüssigkeitsspender 90 zur Abgabe einer nikotin- oder cannabishaltigen Flüssigkeit. Die Austragvorrichtung 100 verfügt über ein lippenbetätigbares Austragventil 120. Drückt der Nutzer mit seiner Lippe oder seinen Zähnen auf die Betätigungsfläche 122, so wird ein Durchströmen des Schlauchabschnitts 121 ermöglicht und die Flüssigkeit strömt in zerstäubter Form durch die Austragdüseneinrichtung 140 hinaus.

Die Fig. 15 und 16 zeigen die Bauweise einer grundsätzlich anders gestalteten Flüssigkeitskartusche 10. Wie sich anhand der Fig. 16 ersehen lässt, besteht diese aus nur drei Teilen, nämlich zwei Wandungsbauteilen 20A, 20B sowie einem Beutel 60, der einen Beutelkörper 62 in Art einer Tube sowie ein einstückig damit verbundenes Austrittsventilbauteil 40 aufweist. Das Austrittsventilbauteil 40 verfügt über einen zentralen Pin, der an einer konischen Innenfläche des Wandungsbauteils 20A anliegt und hierdurch das Austrittventil 32 in einem geschlossenen Zustand hält.

Wie anhand der Fig. 15 zu ersehen ist, ist bei einer solchen Gestaltung der Flüssigkeitsraum 26 durch den Innenraum der Tube 60 gebildet, während der Zwischenraum zwischen der dem Beutel 60 und der Innenseite des aus den Wandungsbauteilen 20A, 20B zusammengesetzten Außenkörpers 20 den Gasraum 24 bildet.

Bei der Gestaltung gemäß Fig. 15 und 16 ist vorgesehen, dass der Gasraum 24 mit Gas unter hohem Druck gefüllt wird, bevor die Wandungsbauteile 20A, 20B miteinander verbunden werden, insbesondere miteinander verschweißt oder verklebt werden.

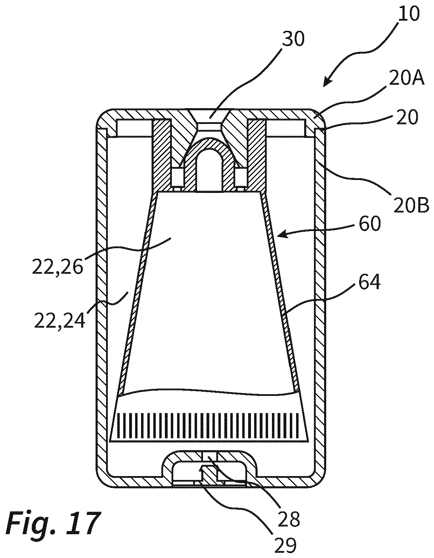

Bei der Ausgestaltung gemäß Fig. 17 ist hiervon abweichend vorgesehen, dass das Wandungsbauteil 20B über eine Gasbefüllungsöffnung 28 verfügt, die nach der Gasbefüllung mittels eines Stopfenbauteils 29 zu verschließen ist.

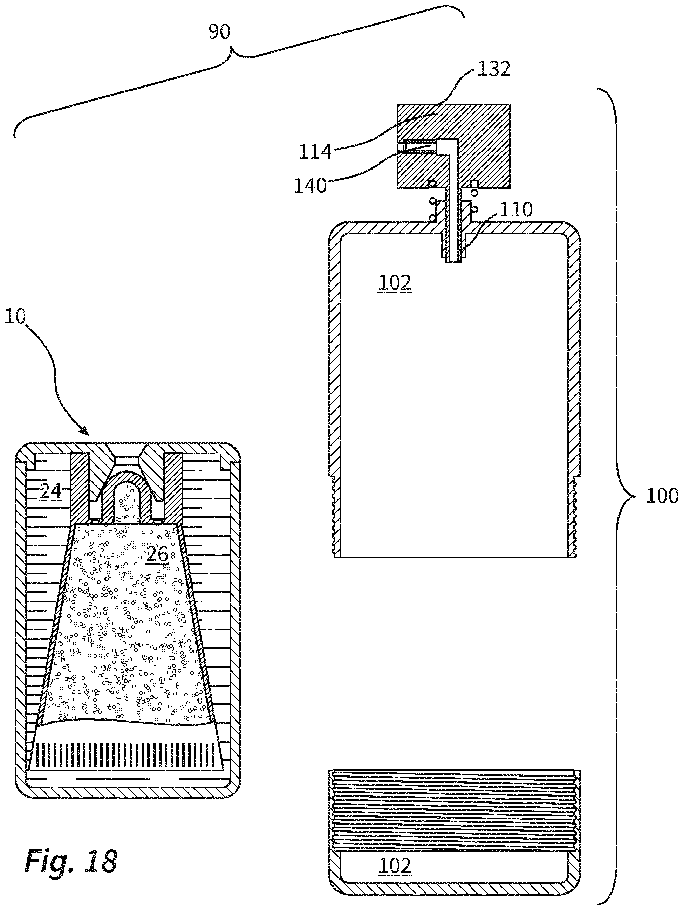

Die Fig. 18 zeigt wiederum die Flüssigkeitskartusche 10 der Fig. 15 und 16 und eine hieran angepasste Austragvorrichtung 100.

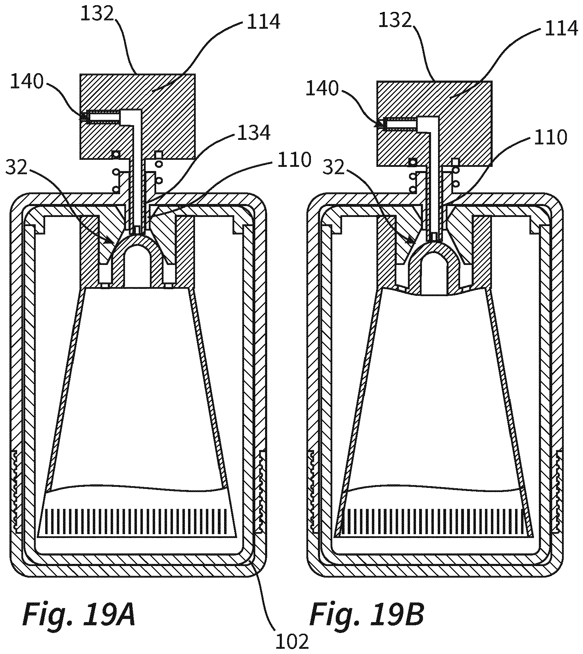

Wie anhand der Fig. 19A und 19B zu ersehen ist, wird die Flüssigkeitskartusche 10 in einen Aufnahmeraum 102 der Austragvorrichtung 100 eingesetzt, wobei im Zuge des Zuschraubens der Austragvorrichtung 100 ein Führungsstutzen der Austragvorrichtung 100 in die Austrittsöffnung 30 eindringt, hierbei jedoch noch keine Verformung oder ein Öffnen des Austrittventilbauteils 40 verursacht. Erst wenn ausgehend von dem insoweit fertiggestellten Zustand der Fig. 19A der Betätigungsdrücker 114 über seine Betätigungsfläche 132 niedergedrückt wird, wird ein hiermit verbundener und durch den Führungsstutzen geführter Öffnungsstutzen 134 tiefer in die Austrittsöffnung 30 eingeschoben, so dass er in Kontakt mit dem Austrittventil 32 gelangt und dieses öffnet. Hierdurch wird der Flüssigkeitsaustrag eingeleitet. Sobald der Betätigungsdrücker 114 in seine Ausgangsstellung zurückkehrt, kann das Austrittsventil 32 wieder schließen, so dass der Flüssigkeitsaustrag endet.

Bei der Gestaltung der Fig. 19A, 19B ist somit abweichend von der Gestaltung der Fig. 13A, 13B vorgesehen, dass das Austrittventil 32 der Flüssigkeitskartusche 10 das einzige Ventil ist und dieses fallweise geöffnet wird, wenn der Benutzer durch Niederdrücken der Betätigungsfläche 132 Flüssigkeit austragen möchte.

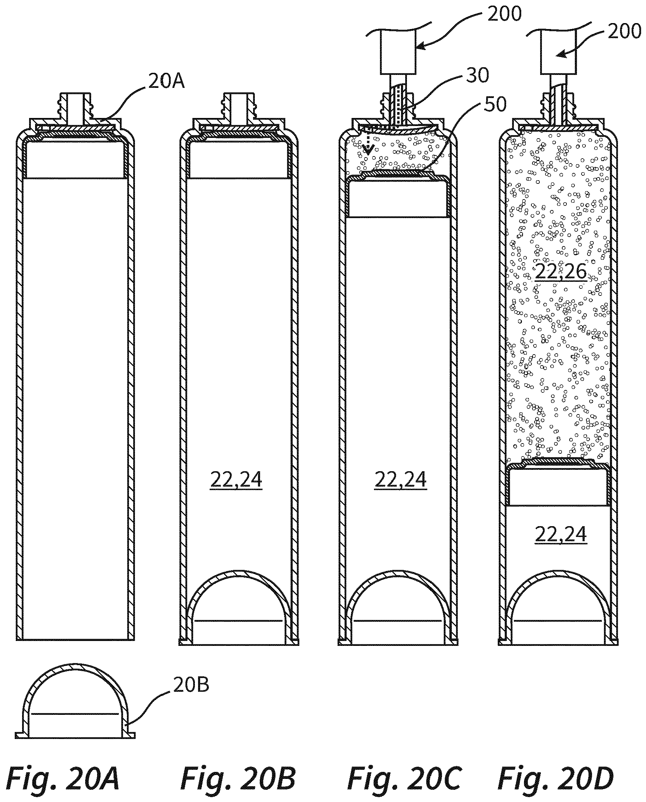

Die Fig. 20A bis 23D zeigen verschiedene Arten der Befüllung der vorbeschriebenen Flüssigkeitskartuschen.

Bei dem Befüllungsvorgang gemäß Fig. 20A ist vorgesehen, dass das Wandungsbauteil 20B vor der Befüllung zunächst nicht mit dem Wandungsbauteil 20A verbunden wird. Stattdessen wird durch das untere und noch offene Ende des Wandungsbauteils 20A und die dort vor Fügung der Wandungsbauteile 20A, 20B vorgesehene Öffnung Gas in den Druckraum 22 geleitet und dann in der durch Fig. 20B verdeutlichten Weise das Wandungsbauteil 20B mit dem Wandungsbauteil 20A verbunden, bspw. verschweißt, insbesondere per Reibschweißung. In diesem Zustand der Fig. 20B befindet sich der Druckraum 22 somit bereits unter Überdruck, üblicherweise unter einem Überdruck von 2 bis 3 bar. Anschließend wird die Flüssigkeitskartusche 10 bestimmungsgemäß an den Befüller geliefert, welcher dann durch die Austrittsöffnung 30 hindurch Flüssigkeit mittels eines Füllstutzens 200 in den Flüssigkeitsraum 26 einströmen lässt, wie in Fig. 20C gezeigt. Die Flüssigkeit drückt den Trennkolben 50 nach unten, bis der vollständig befüllte Zustand der Fig. 20D erreicht ist und die Befüllung beendet ist.

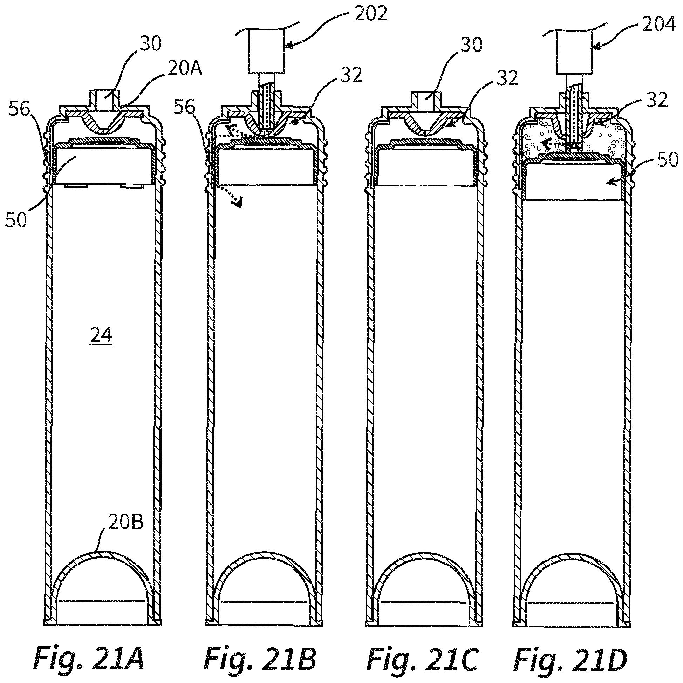

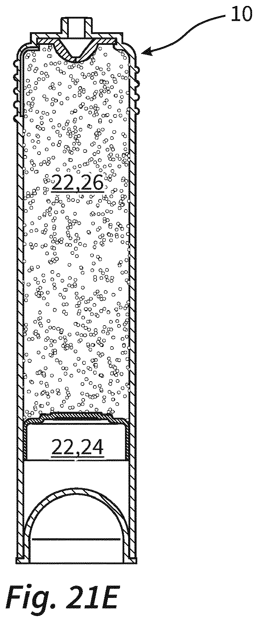

Bei der Gestaltung gemäß den Fig. 21A bis 21D ist vorgesehen, dass die Wandungsbauteile 20A, 20B vor Befüllung bereits fest und gasdicht miteinander verbunden sind. Zur Befüllung wird in der durch Fig. 21B verdeutlichten Weise zunächst ein erster Füllstutzen 202 durch die Austrittsöffnung 30 eingeschoben, der das hier vorgesehene Austrittventil 32 öffnet und Gas in den Druckraum 22 strömen lässt. Dieses Gas kann durch einen Umgehungskanal 56 aus dem noch nicht befüllten Flüssigkeitsraum 26 in den Gasraum 24 strömen. Sobald der gewünschte Gasdruck von etwa 2 bis 3 erreicht ist, wird der erste Füllstutzen 202 aus der Austrittsöffnung 30 herausgezogen und in der durch Fig. 21D verdeutlichten Weise ein zweiter Füllstutzen 204 in die Austrittsöffnung 30 eingeschoben, welcher soweit eingeschoben wird, dass er durch das Austrittsventil 32 hindurchgeschoben wird, in Kontakt mit dem Trennkolben 50 gelangt und diesen aus der Ausgangsstellung weiter nach unten verschiebt, bis dieser über das Ende des Umgehungskanals 56 hinausgeschoben ist. Diese Lage des Trennkolbens 50 ist in Fig. 21C dargestellt. Nun beginnt die Befüllung mit Flüssigkeit, wobei die Flüssigkeit am Trennkolben 50 vorbei nicht in relevantem Maße in den Gasraum 24 gelangen kann. Ist der Zustand der Fig. 21E erreicht, so ist der Befüllungsvorgang abgeschlossen.

Die Fig. 22A bis 22D zeigen eine weitere Variante des Befüllungsvorgangs. Hier ist am Trennkolben 50 ein Abstandshalter 55 vorgesehen. Das Verfahren sieht vor, dass wiederum ausgehend von der Flüssigkeitskartusche 10, deren Wandungsbauteile 20A, 20B bereits gasdicht miteinander verbunden sind, zunächst mittels eines ersten Füllstutzens 202 Gas durch die Austrittsöffnung 30 in den Druckraum 22 geleitet wird. Dieses Gas gelangt zunächst in den Flüssigkeitsraum 26 und verschiebt den Trennkolben 50 bis in eine untere Endlage, in der jedoch der Gasraum 24 noch ein erhebliches Volumen aufweist, da der Abstandshalter 55 ein Weiterschieben des Trennkolbens 50 verhindert. Dieser Zustand ist in Fig. 22B dargestellt. Der Gasüberdruck im Flüssigkeitsraum 26 bewirkt, dass Gas am Trennkolben vorbei bis in den Gasraum 24 strömt.

In der in Fig. 22C verdeutlichten Weise wird dann in einer Überkopflage mittels eines zweiten Füllstutzens 204 Flüssigkeit von unten in den Flüssigkeitsraum 26 gedrückt. Diese Flüssigkeit bewirkt, dass das im Flüssigkeitsraum 26 zu diesem Zeitpunkt noch befindliche Gas ebenfalls am Trennkolben 50 vorbei in den Gasraum 24 gedrückt wird. Sobald der Zustand der Fig. 22D erreicht ist, ist der Befüllungsvorgang abgeschlossen.

Es hat sich gezeigt, dass auch nach Rücküberführung der Flüssigkeitskartusche 10 in ihre Normallage mit nach oben weisen der Austrittsöffnung 30 keine Vermengung zwischen dem Gas im Gasraum 24 und der Flüssigkeit im Flüssigkeitsraum 26 in relevantem Maße stattfindet. Dies wird unter anderem dadurch verhindert, dass der Gasdruck im Gasraum 24 den napfartigen Trennkolben 50 aufweitet und somit den Spalt zwischen dem Trennkolben und der Mantelwand des Wandungsbauteils 21 hierdurch schließt.

Die Fig. 23A bis 23D zeigen die Befüllung im Falle der Flüssigkeitskartusche der Fig. 17. Hier ist vorgesehen, dass ausgehend von der weder mit Gas noch mit Flüssigkeit befüllten Flüssigkeitskartusche der Fig. 23A zunächst mittels eines ersten Füllstutzens 202 die Tube, also der Flüssigkeitsraum 26, befüllt wird, wobei der Füllstutzen 202 hierzu das Austrittventil 32 mechanisch öffnet. Fig. 23B zeigt die Flüssigkeitsbefüllung bei geöffnetem Austrittsventil 32.

Anschließend wird in der in Fig. 23C verdeutlichten Weise im Bereich der Gasbefüllungsöffnung 28 ein zweiter Füllstutzen angesetzt, der Gas in den Gasraum 24 drückt. Sobald der gewünschte Gasdruck erzielt wird, wird mittels eines Schließstiftes das bereits zur Fig. 17 beschriebene Stopfenbauteil 29 in die Gasbefüllungsöffnung 28 hineingedrückt, so dass das Gas hierdurch nicht mehr entweichen kann.

## Patentansprüche

1. Flüssigkeitskartusche (10) zur Aufnahme von unter Druck stehender Flüssigkeit mit den folgenden Merkmalen:
a. die Flüssigkeitskartusche (10) weist einen aus mindestens zwei Wandungsbauteilen (20A, 20B) zusammengesetzten Außenkörper (20) auf, der einen Druckraum (22) zur Aufnahme von unter Druck stehendem Gas und unter Druck stehender Flüssigkeit umgibt, und
b. die Flüssigkeitskartusche (10) weist eine Austrittsöffnung (30) auf, die eines der Wandungsbauteile (20A) durchdringt, und
c. die Flüssigkeitskartusche (10) weist ein Austrittsventilbauteil (40) auf, welches an einer Innenseite des Druckraums (22) an mindestens einem Wandungsbauteil (20A) beweglich gehalten oder befestigt ist und welches in einem geschlossenen Zustand den Austritt von Flüssigkeit aus dem Druckraum (22) durch die Austrittsöffnung (30) unterbindet, wobei das Austrittsventilbauteil (40) durch Kraftbeaufschlagung von außen in einen geöffneten Zustand überführt werden kann, und
d. der Druckraum (22) beinhaltet eine verlagerbare oder verformbare Trennwand (52,64), die den Druckraum (22) in einen Gasraum (24) und einen Flüssigkeitsraum (26) unterteilt.

2. Flüssigkeitskartusche (10) nach Anspruch 1 mit dem folgenden weiteren Merkmal:
a. das Austrittsventilbauteil (40) schließt durch Anlage an einer Ventilgegenfläche (42) an einem der Wandungsbauteile, vorzugsweise an jenem Wandungsbauteil (20A), welches von der Austrittsöffnung (30) durchdrungen ist.

3. Flüssigkeitskartusche (10) nach Anspruch 1 mit dem folgenden weiteren Merkmal:
a. das Austrittsventilbauteil (40) ist von einer Ventilöffnung durchbrochen, wobei an die Ventilöffnung angrenzende Ränder in einem geschlossenen Zustand des Austrittventilbauteils (40) aneinander anliegen und im geöffneten Zustand voneinander zumindest partiell beabstandet sind,
vorzugsweise mit mindestens einem der folgenden zusätzlichen Merkmale:
b. das Austrittsventilbauteil (40) weist in Richtung des Flüssigkeitsraums weisende Druckflächen auf, die durch Druckbeaufschlagung der Flüssigkeit aufeinander zu gedrückt werden und hierdurch die an die Ventilöffnung angrenzenden Ränder zusammendrücken, und/oder
c. das Austrittsventilbauteil (40) weist einen in den Flüssigkeitsraum hineinragenden Schlauchabschnitt auf, und/oder
d. die Ventilöffnung ist als Ventilschlitz ausgebildet, wobei vorzugsweise der Schlitz an einer domartig gewölbten Struktur vorgesehen ist, und/oder
e. an einer vom Flüssigkeitsraum weg weisenden Seite des Austrittsventilbauteils (40) ist eine Aufweitungsstuktur vorgesehen, mittels derer ein einfahrender Füllstutzen die Ventilöffnung aufweiten kann.

4. Flüssigkeitskartusche (10) nach einem der vorstehenden Ansprüche mit dem folgenden weiteren Merkmal:
a. die Trennwand (52) wird durch einen Trennkolben (50) gebildet, der an einer Innenseite des Außenkörpers (20) entlanggleitend beweglich ist, wobei auf der der Austrittsöffnung (30) abgewandten Seite des Trennkolbens (50) der Gasraum (24) vorgesehen ist und auf der der Austrittsöffnung zugewandten Seite des Trennkolbens der Flüssigkeitsraum (26) vorgesehen ist,
vorzugsweise mit mindestens einem der folgenden zusätzlichen Merkmale:
b. der Trennkolben (50) weist eine zum Gasraum offenen napfartige Struktur auf, deren Seiten durch eine Mantelfläche des Trennkolbens gebildet werden, und/oder
c. der Trennkolben (50) weist eine asymmetrische Gestaltung auf, die bei Überdruck im Gasraum gegenüber dem Flüssigkeitsraum eine radiale Aufweitung des Trennkolbens (50) bewirkt, und/oder
d. an der Innenseite des Außenkörpers (20) ist ein Umströmungskanal vorgesehen, der bei Anordnung des Trennkolbens (50) im Bereich des Umströmungskanals ein Umströmen des Trennkolbens (50) ermöglicht, und/oder
e. der Trennkolben (50) und/oder eine bodenseitige Stirnwand (21C) des Außenkörpers und/oder eine Mantelfläche des Außenkörpers ist mit einer Anschlagsfläche und/oder einem Abstandshalter versehen, mittels dessen ein Mindestabstand zwischen der bodenseitigen Wandung und dem Trennkolben (50) gewährleistet wird.

5. Flüssigkeitskartusche (10) nach einem der Ansprüche 1 bis 3 mit dem folgenden weiteren Merkmal:
a. die Trennwand (64) wird durch einen Beutelkörper (62) und dessen flexibler Beutelwand (64) gebildet, wobei innenseitig der Beutelwand (64) der Flüssigkeitsraum (26) und zwischen der Beutelwand (64) und dem Außenkörper (20) der Gasraum (24) vorgesehen ist,
vorzugsweise mit dem folgenden zusätzlichen Merkmal:
b. der Beutelkörper (62) und das Austrittsventilbauteil (40) sind einstückig ausgebildet.

6. Flüssigkeitskartusche (10) nach einem der vorstehenden Ansprüche mit den folgenden weiteren Merkmalen:
a. das Austrittsventilbauteil (40) ist als Ventilplatte (40) ausgebildet, und
b. die Ventilplatte (40) ist durch einen von außen einschiebbaren Öffnungsstift oder Öffnungsstutzen (110) auslenkbar, so dass die Ventilplatte ihren geöffneten Zustand einnimmt,
vorzugsweise mit mindestens einem der folgenden zusätzlichen Merkmale:
c. die Ventilplatte (40) weist im unbelasteten Zustand eine plane Formgebung auf, und/oder
d. die Ventilplatte (40) ist außenseitig an einer Innenseite des Außenkörpers festgelegt, vorzugsweise mittels einer Schnappverbindung (23), und/oder
e. die Ventilplatte (40) weist mindestens eine Durchbrechung (41) auf, und/oder
f. die Ventilplatte weist einen außenseitigen Rand auf, der umlaufend an der Innenseite des Außenkörpers anliegt, oder
g. die Ventilplatte weist einen außenseitigen Rand auf, in dem mindestens eine außenseitige Einbuchtungen vorgesehen ist, im Bereich derer der Rand von der Innenseite des Außenkörpers beabstandet ist, und/oder
h. an einer Innenseite des Außenkörpers (20) ist die Austrittsöffnung (30) umgebend ein in Richtung der Ventilplatte weisender umlaufender Anlagesteg (42) als Ventilgegenfläche (42) vorgesehen, an dem die Ventilplatte (40) im geschlossenen Zustand anliegt, und/oder
i. die Ventilplatte (40) ist als Stanzteil hergestellt, insbesondere als Stanzteil aus Polyethylen (PE) oder thermoplastischem Elastomer (TPE).

7. Flüssigkeitskartusche (10) nach einem der vorstehenden Ansprüche mit den folgenden weiteren Merkmalen:
a. ein erstes Wandungsbauteil (20A) umfasst einstückig eine Mantelwand (21A) sowie eine erste Stirnwand (21B), wobei der ersten Stirnwand (21B) die Austrittsöffnung (30) vorgesehen ist, und
b. ein zweites Wandungsbauteil (20B) bildet eine der Austrittsöffnung (30) gegenüberliegende bodenseitige Stirnwand (21C),
vorzugsweise mit dem folgenden zusätzlichen Merkmal:
c. das erste Wandungsbauteil (20A) bildet mit seiner Innenseite eine zylindrische Gleitfläche, entlang derer der Trennkolben (50) entlang gleitet.

8. Flüssigkeitskartusche (10) nach einem der vorstehenden Ansprüche mit dem folgenden weiteren Merkmal:
a. die Wandungsbauteile (20A, 20B), die Trennwand (52, 64) und das Austrittsventilbauteil (40) bestehen aus Materialen, die in einem gemeinsamen Recyclingprozess recyclebar sind,
vorzugsweise mit mindestens einem der folgenden zusätzlichen Merkmale:
b. die Flüssigkeitskartusche (10) besteht zum überwiegenden Teil aus einem Polyethylenterephthalat (PET), wobei vorzugsweise mindestens 60% der Masse der Flüssigkeitskartusche (10) aus PET besteht, insbesondere vorzugsweise mindestens 75%, und/oder
c. die Flüssigkeitskartusche (10) besteht zum überwiegenden Teil aus Materialien aus der Gruppe umfassend Polyethylenterephthalate (PET), Polyethylene (PE), Polypropylene (PP), wobei vorzugsweise mindestens 75% der Masse der Flüssigkeitskartusche (10) aus diesen Materialien besteht, insbesondere vorzugsweise mindestens 90%.

9. Flüssigkeitskartusche (10) nach einem der vorstehenden Ansprüche mit dem folgenden weiteren Merkmal:
a. die Wandungsbauteile (20A, 20B), der Trennkolben (50) bzw. der Beutel (60) sowie das Austrittsventilbauteil (40) weisen jeweils ein Material aus der Gruppe umfassend Polyethylenterephthalate (PET), Polyethylene (PE), Polypropylene (PP) und thermoplastische Elastomere (TPE) auf,
vorzugsweise mit mindestens einem der folgenden zusätzlichen Merkmale:
b. die Wandungsbauteile (20A, 20B) bestehen aus PET, und/oder
c. der Trennkolben (50) oder der Beutel (60) besteht aus PE, und/oder
d. das Austrittsventilbauteil (40) besteht aus TPE oder aus PE, und/oder
e. der Beutelkörper (62) und das Austrittsventilbauteil (40) sind einstückig ausgebildet und bestehen aus PE.

10. Flüssigkeitskartusche (10) nach einem der vorstehenden Ansprüche mit einem der folgenden weiteren Merkmale:
a. der Flüssigkeitsraum (26) ist mit einer nikotin- und/oder cannabishaltigen Flüssigkeit befüllt, oder
b. der Flüssigkeitsraum (26) ist mit einer pharmazeutischen Flüssigkeit befüllt, oder
c. der Flüssigkeitsraum (26) ist mit einer Flüssigkeit zur Körperhygiene befüllt, insbesondere mit einer Deodorant-Flüssigkeit oder mit einem Parfum, und/oder
d. der Flüssigkeitsraum ist mit einer hochviskosen Flüssigkeit befüllt, vorzugsweise einer Hautpflegecreme oder Zahnpasta.

11. Flüssigkeitskartusche nach einem der vorstehenden Ansprüche mit mindestens einem der folgenden zusätzlichen Merkmale:
a. an einer Außenseite des Außenkörpers (20) ist ein Außengewinde (70) oder ein Bajonettzur Ankopplung an eine Austragvorrichtung vorgesehen, und/oder
b. der Beutel (60) ist in Art einer Tube (60) ausgestaltet, und/oder
c. das Austrittsventilbauteil weist eine durch die Austrittsöffnung hindurch zugängliche Betätigungsfläche (43) auf, die durch Kraftbeaufschlagung eine Verlagerung des Austrittventilbauteils (40) in den geöffneten Zustand bewirkt, oder
d. das Austrittsventilbauteil weist einen durch die Austrittsöffnung hindurch nach außen ragenden Betätigungsabschnitt mit einer von außen zugänglichen Betätigungsfläche auf, die durch Kraftbeaufschlagung eine Verlagerung des Austrittventilbauteils (40) in den geöffneten Zustand bewirkt, und/oder
e. der Flüssigkeitsraum weist ein Volumen von maximal 118 ml auf, und/oder
f. mindestens zwei der Wandungsbauteile sind miteinander über eine Schweißverbindung verbunden, wobei die Schweißverbindung vorzugsweise eine Laserschweißverbindung, eine Reibschweißverbindung oder eine Ultraschallschweißverbindung ist, und/oder
g. mindestens zwei der Wandungsbauteile sind unter Bildung einer Presspassung miteinander verbunden, wobei hierfür vorzugsweise eine Kopplungseinrichtung vorgesehen ist, die eine Gewindeverbindung umfasst und wobei vorzugsweise mindestens eines der Wandungsbauteile mit einer konischen Aufweitung oder Einschnürung versehen ist, um die Presspassung beim Einschrauben zu erzielen, und/oder
h. mindestens zwei der Wandungsbauteile sind über eine Klebeverbindung miteinander verbunden, und/oder
i. in mindestens einem der Wandungsbauteile ist eine Gasbefüllungsöffnung vorgesehen, die in den Gasraum mündet, wobei das Wandungsbauteil eine Ventileinrichtung aufweist, die durch Überdruck im Gasraum geschlossen wird, und/oder
j. das Auslassventilbauteil ist als in einem Ventilraum frei bewegliches Auslassventilbauteil ausgebildet, vorzugsweise in Form eines Kugelkörpers, insbesondere aus TPE, wobei das Auslassventil in einer durch Überdruck im Flüssigkeitsraum verursachten Endlage eine Ventilöffnung verschließt, und/oder
k. mindestens eines der Wandungsbauteile ist aus einem transparenten Kunststoff gefertigt, und/oder
l. in einem der Wandungsbauteile ist eine in den Gasraum mündende Gasbefüllungsöffnung vorgesehen, wobei die Gasbefüllungsöffnung vorzugsweise mit einem den Gasaustritt aus dem Druckraum unterbindenden Ventil oder einem Stopfenbauteil versehen ist, und/oder
m. die Flüssigkeitskartusche (10) weist genau zwei Wandungsbauteile (20A, 20B) auf, aus denen der Außenkörper (20) zusammengesetzt ist.

12. Flüssigkeitsspender (90) zum Austrag von Flüssigkeit mit dem folgenden Merkmal:
a. der Flüssigkeitsspender (90) weist eine Austragvorrichtung (100) und eine an oder in der Austragvorrichtung (100) befestigte Flüssigkeitskartusche (10) auf,
**gekennzeichnet durch** das folgende weitere Merkmal:
b. die Flüssigkeitskartusche (10) ist nach einem der Ansprüche 1 bis 11 ausgebildet.

13. Flüssigkeitsspender nach Anspruch 12 mit dem folgenden zusätzlichen Merkmal:
a. die Austragvorrichtung (100) weist einen Aufnahmeraum (102) zur Aufnahme der Flüssigkeitskartusche (10) auf,
vorzugsweise mit mindestens einem der folgenden zusätzlichen Merkmale:
b. der Aufnahmeraum (102) ist von einer Mantelfläche (104) abschnittsweise begrenzt, an deren Innenseite ein Innengewinde (106) vorgesehen ist, wobei korrespondierend hierzu an einer Außenseite des Außenkörpers ein Außengewinde (70) vorgesehen ist, und/oder
c. derAufnahmeraum (102) ist von einer Mantelfläche (104) abschnittsweise begrenzt, die aus einem transparenten Kunststoff besteht.

14. Flüssigkeitsspender (90) nach einem der Ansprüche 12 oder 13 mit dem folgenden zusätzlichen Merkmal:
a. die Austragvorrichtung (100) weist zum Öffnen des Austrittsventils einen Öffnungsstift oder einen Öffnungsstutzen (110) auf, der durch die Austrittsöffnung (30) in die Flüssigkeitskartusche (10) hineinragt,
vorzugsweise mit den folgenden zusätzlichen Merkmalen:
b. die Austragvorrichtung (100) weist zusätzlich zum Austrittsventilbauteil (40) ein mittels einer Betätigungsfläche (122) manuell schaltbares Austragventil (120) auf, und/oder
c. der Öffnungsstutzen (110) weist eine hülsenförmige Form auf, wobei die Außenseite des Öffnungsstutzens (110) vorzugsweise einen Durchmesser aufweist, der dichtend an einer Innenseite der Austrittsöffnung (30) anliegt.

15. Flüssigkeitsspender (90) nach einem der Ansprüche 12 bis 14 mit dem folgenden zusätzlichen Merkmal:
a. die Austragvorrichtung (100) weist eine Betätigungsfläche (132) zur manuellen Betätigung auf, wobei die Betätigungsfläche (132) mit einem Öffnungsstift oder Öffnungsstutzen (134) gekoppelt ist, der bei Betätigung eine Kraftbeaufschlagung und damit ein Öffnen des Austrittsventilbauteils (40) bewirkt.

16. Flüssigkeitsspender (90) nach einem der Ansprüche 12 bis 15 mit dem folgenden zusätzlichen Merkmal:
a. die Austragvorrichtung (100) weist eine Austragdüseneinrichtung (140) auf, insbesondere mit dem folgenden zusätzlichen Merkmal:
b. die Austragdüseneinrichtung (140) weist eine Mehrzahl von Austrittsdüsenöffnungen auf.

17. Verfahren zur Befüllung einer Flüssigkeitskartusche (10) nach einem der Ansprüche 1 bis 11 mit dem folgenden Merkmal:
a. es wird in beliebiger Reihenfolge Gas in den Gasraum und Flüssigkeit in den Flüssigkeitsraum eingebracht.

18. Verfahren nach Anspruch 17 mit dem folgenden weiteren Merkmal:
a. das Gas wird durch eine von der Austrittsöffnung abweichende Öffnung in den Gasraum einbracht,
insbesondere vorzugsweise mit einem der folgenden weiteren Merkmale:
b. das Gas wird durch eine Öffnung eines Wandungsbauteils in den Gasraum eingebracht, bevor die Öffnung durch Verbindung des Wandungsbauteils mit einem zweiten der Wandungsbauteile verschlossen wird, insbesondere mittels einer Schweißverbindung, einer Klebeverbindung oder einer Presspassung, oder
c. das Gas wird durch eine Gasbefüllungsöffnung in den Gasraum eingebracht, insbesondere nach mechanischem oder druckbedingtem Öffnen eines Gasbefüllungsventils oder vor Verschließen der Gasbefüllungsöffnung mittels eines Stopfenbauteils.

19. Verfahren nach Anspruch 17 mit dem folgenden weiteren Merkmal:
a. sowohl das Gas als auch die Flüssigkeit werden nacheinander durch die Austrittsöffnung (30) hindurch in den Druckraum (22) eingebracht,
vorzugsweise mit mindestens einem der folgenden zusätzlichen Merkmale:
b. zunächst wird das Gas in den Druckraum eingebracht, wobei es in den Flüssigkeitsraum und in den Gasraum gelangt, und anschließend wird in einer Lage der Flüssigkeitskartusche mit unten angeordnetem Flüssigkeitsraum die Flüssigkeit von unten in den Flüssigkeitsraum eingebracht, wobei zumindest ein Teil des Gases aus dem Flüssigkeitsraum in den Gasraum verdrängt wird, und/oder
c. der Trennkolben zwischen dem Flüssigkeitsraum und dem Gasraum befindet sich zu Beginn der Einbringung des Gases in einer Haltestellung, aus der er mittels einer Druckstoßes oder mittels eines Werkzeugs durch die Austrittsöffnung hindurch während der Einbringung gelöst wird.
